(19) Europäisches Patentamt
European Patent Office
Office européen des brevets

(11) **EP 1 215 497 B1**

(12) # EUROPEAN PATENT SPECIFICATION

(45) Date of publication and mention of the grant of the patent:
**07.01.2009 Bulletin 2009/02**

(51) Int Cl.:
*G01N 33/543* (2006.01) *G01N 33/569* (2006.01)
*A61K 39/395* (2006.01) *C07K 16/28* (2006.01)

(21) Application number: **01127425.5**

(22) Date of filing: **16.12.1997**

(54) **Diagnostics for transmissible spongiform encephalopathy**

Diagnostik für übertragbare spongiforme Enzephalopathie

Diagnostique pour l'encéphalopathie spongiforme transmissible

(84) Designated Contracting States:
**AT BE CH DE DK ES FI FR GB GR IE IT LI LU MC NL PT SE**

(43) Date of publication of application:
**19.06.2002 Bulletin 2002/25**

(62) Document number(s) of the earlier application(s) in accordance with Art. 76 EPC:
**97122186.6 / 0 931 551**

(73) Proprietor: **UNIVERSITY OF ZURICH**
**8600 Zürich (CH)**

(72) Inventors:
- **Aguzzi, Adriano**
**8001 Zürich (CH)**
- **Klein, Michael A.**
**53925 Kall (DE)**
- **Raeber, Alex**
**8057 Zürich (CH)**
- **Weissmann, Charles**
**8053 Zürich (CH)**
- **Zinkernagel, Rolf**
**8126 Zumikon (CH)**

(74) Representative: **Modiano, Micaela Nadia et al**
**Modiano Josif Pisanty & Staub Ltd**
**Thierschstrasse 11**
**80538 München (DE)**

(56) References cited:
**WO-A-89/12458** **WO-A-97/10505**

- **BENDHEIM ET AL.: "Nearly ubiquitous tissue distribution of the scrapie agent precursor protein" NEUROLOGY, vol. 42, January 1992 (1992-01), pages 149-156, XP002065592**
- **CASHMAN ET AL.: "Cellular isoform of the scrapie agent protein participates in lymphocyte activation" CELL, vol. 61, 6 April 1990 (1990-04-06), pages 185-192, XP002066608**
- **LASM1ZAS ET AL.: "Immune system-dependent and -independent replication of the scrapie agent" JOURNAL OF VIROLOGY, vol. 70, no. 2, February 1996 (1996-02), pages 1292-1295, XP002066609**
- **EKLUND ET AL.: "Pathogenesis of scrapie virus infection in the mouse" THE JOURNAL OF INFECTIOUS DISEASES, vol. 117, 1967, pages 15-22, XP002065593**
- **BÜELER ET AL.: "Normal development and behaviour of mice lacking the neuronal cell-surface PrP protein" NATURE, vol. 356, 16 April 1992 (1992-04-16), pages 577-582, XP002065595**
- **DIOMEDE L ET AL: "Activation effects of a prion protein fragment [PrP-(106-126)] on human leucocytes." BIOCHEMICAL JOURNAL, (1996 DEC 1) 320 ( PT 2) 563-70. JOURNAL CODE: 9YO. ISSN: 0264-6021., XP002081288 ENGLAND: United Kingdom**
- **WILLIAMSON R A ET AL: "Circumventing tolerance to generate autologous monoclonal antibodies to the prion protein" PROCEEDINGS OF THE NATIONAL ACADEMY OF SCIENCES OF USA, NATIONAL ACADEMY OF SCIENCE. WASHINGTON, US, vol. 93, July 1996 (1996-07), pages 7279-7282, XP002924866 ISSN: 0027-8424**
- **MILLSON ET AL.: "Early distribution of radioactive liposomes and scrapie infectivity in mouse tissues following administration by different routes" VETERINARY MICROBIOLOGY, vol. 4, no. 2, 1979, pages 89-99, XP002081287**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 215 497 B1

- KIMBERLIN ET AL.: "Pathogenesis of mouse scrapie: Dynamics of agent replication in spleen, spinal cord and brain after infection by different routes" JOURNAL OF COMPARITIVE PATHOLOGY, vol. 89, no. 4, 4 October 1979 (1979-10-04), pages 551-562, XP002081286
- CAUGHEY B ET AL: "Detection of prion protein mRNA in normal and scrapie -infected tissues and cell lines." JOURNAL OF GENERAL VIROLOGY, (1988 MAR) 69 ( PT 3) 711-6. JOURNAL CODE: I9B. ISSN: 0022-1317., XP002081289 ENGLAND: United Kingdom
- KLEIN ET AL.: "A crucial role for B cells in neuroinvasive scrapie" NATURE, vol. 390, 16 - 18 December 1997, pages 687-690, XP002065601
- BLÄTTLER ET AL.: "PrP-expressing tissue required for transfer of scrapie infectivity from spleen to brain" NATURE, vol. 389, 4 September 1997 (1997-09-04), page 69-73 XP002065594
- O'ROURKE ET AL.: "SCID mouse spleen does not support scrapie agent replication" JOURNAL OF GENERAL VIROLOGY, vol. 75, 1994, pages 1511-1514, XP002066607
- FIELD E J ET AL: "Cellular sensitization in kuru, Jakob-Creutzfeldt disease and multiple sclerosis: with a note on the biohazards of slow infection work." ACTA NEUROLOGICA SCANDINAVICA, (1975 APR) 51 (4) 299-309. JOURNAL CODE: 1BS. ISSN: 0001-6314., XP002081290 Denmark
- MAYER R J ET AL: "The role of protein ubiquitination in neurodegenerative disease." ACTA BIOLOGICA HUNGARICA, (1991) 42 (1-3) 21-6. REF: 24 JOURNAL CODE: 0DF. ISSN: 0236-5383., XP002081291 Hungary
- FOLEY G L ET AL: "Rabies-induced spongiform change and encephalitis in a heifer." VETERINARY PATHOLOGY, (1995 MAY) 32 (3) 309-11. JOURNAL CODE: XBQ. ISSN: 0300-9858., XP002081292 United States

## Description

The present invention relates to diagnostics of transmissible spongiform encephalopathy.

## Background art

Transmissible spongiform encephalopathies (TSE's) comprise a group of slow degenerative diseases of the CNS such as Creutzfeld-Jakob disease (CJD), new variant CJD (termed vCJD)[1,1], Gerstmann-Sträussler-Scheinker disease (GSS) and kuru in man and scrapie in sheep or BSE (mad cow disease) in cattle.

The occurrence of these exotic illnesses is still fortunately very low, probably occuring at 1:1,000,000 but there are striking similarities when compared to the Alzheimer-syndrome. However, BSE is now reported to have reached epidemic proportions in England and is caused by the use of rendered materials in cattle feed and can originate with scrapie infected sheep. Dairy cattle in particular are at the highest measurable risk. A tragically similar incidence has occurred with humans.

During the production of human growth hormone from human glands collected from cadavers, the pathogenic agent of Creutzfeld-Jacob Syndrome was introduced. Several Cases have now been reported in patients treated with this growth hormone. The patients were predominantly children, whereas the disease normally attacks adults over 50 years of age.

These examples point out the potential danger of these new diseases and the difficulties in diagnosing and treating them effectively.

The unusual properties of the pathogenic agent, designated as "prion" (Prusiner, S.B. Novel proteinaceous infectious particles cause scrapie. Science 216, 136-144. (1982)) include the extremely long incubation periods, exceeding one year, and resistance to high temperatures, formaldehyde treatment and UV irradiation (Gordon, W.S. Vet Rec 58,516 (1946); Pattison, I.H. Resistance of the scrapie agent to formalin. J comp Pathol 75, 159-164 (1965); Alper et al., The exceptionally small size of the scrapie agent. Biochem. Biophys. Res. Commun. 22, 278-284 (1966); Latarjet et al., Inactivation of the scrapie agent by near monochromatic ultraviolet light. Nature 227, 1341-1343 (1970)) Speculations arose early on that the scrapie agent might be devoid of nucleic acid (Alper et al., Does the agent of scrapie replicate without nucleic acid? Nature 214, 764-766 (1967); Gibbon, R.A. and Hunter, G.D. Nature of the scrapie agent. Nature 215, 1041-1043 (1967); Pattison, I.H. and Jones, K.M The possible nature of the transmissible agent of scrapie. Vet. Rec. 80, 2-9 (1967)). Considerable evidence now supports the "protein only" hybothesis (Prusiner, S.B. and Hsiao, K.K. Human prion diseases. Ann. Neurol. 35, 385-395 (1994); Weissmann, C. Molecular biology of prion diseases. Trends Cell Biol. 4, 10-14 (1994)) which proposes that the prion is devoid of nucleic acid and identical with $PrP^{SeC}$, a modified form of $PrP^{C}$. $PrP^{C}$ is a normal host protein (Oesch et al., A cellular gene encodes scrapie PrP 27-30 Protein. Cell 40, 735-746 (1985); Chesebro et al., Identification of scrapie prion protein-specific mRNA in scrapie-infected and uninfected brain. Nature 315, 331-333 (1985) found predominantly on the outer surface of neurons, but also in many other tissues (Manson et al., The prion protein gene: a role in mouse embryogenesis? Development 115, 117-122 (1992); Bendheim et al., Nearly ubiquitous tissue distribution of the scrapie agent precursor protein. Neurology 42, 149-156 (1992)). $PrP^{SC}$ is defined as a protease-resistant form of $PrP^{C}$ which readily forms aggregates after detergent treatment (Mc Kinley et al., Scrapie prion rod formation in vitro requires both detergent extraction and limited proteolysis: J. Vitrol. 65, 1340-1351 (1991)). No chemical differences have so far been detected between $PrP^{Se}$ and $PrP^{C}$ (Stahl et al., Structural studies of the scrapie prion protein using mass spectrometry and amino acid sequencing. Biochemistry 32, 1991-2002 (1993)). Prusiner proposed that $PrP^{Se}$, when introduced into a normal cell, causes the conversion of $PrP^{C}$ or its precursor into $PrP^{C}$ (Oesch et al., Search for a scrapie-specific nucleic acid: a progress report. Ciba. Found. Symp. 135, 209-223 (1988);- Prusiner et al., Transgenetic studies implicate interactions between homologous PrP isoforms in scrapie prion replication. Cell 63, 673-686 (1990); Bolton, D.C. and Bendheim, P.E. A modified host protein model of scrapie. Ciba. Found. Symp. 135, 164-181 (1988)). The conversion is believed to result from a conformational rearrangement of $PrP^{C}$. Some researchers still adhere to the virino hypothesis which holds that the infectious agent consists of a nucleic acid genome and the host-derived PrP, which is recruited as some sort of coat (Dickinson, A.G. and Outram, G.W. Genetic aspects of unconventional virus infections: the basis of the virino hypothesis. Ciba. Found. Symp. 135, 63-83 (1988); Hope, J. The nature of the scrapie agent: the evolution of the virino. Ann. N. Y. Acad. Sci. 724, 282-289 (1994)). Finally, the possibility that the infectious agent is a virus with unusual properties is still upheld by some (Diringer et al., The nature of the scrapie agent: the virus theory. Ann. N. Y. Acad. Sci. 724, 246-258 (1994); Pocchiari, M. Prions and related neurological diseases. Molec. Aspects. Med. 15, 195-291 (1994); Rohwer, R.G. The scrapie agent: "a virus by any other name". Curr. Top. Microbiol. Immunol. 172, 195-232 (1991)). No credible evidence for the existence of a scrapie-specific nucleic acid, as demanded by the virus and the virino hyptohesis, has yet been forthcoming (Oesch et al., vide supra; Kellings et al., Futher analysis of nucleic acids in purified scrapie prion preparations by improved return refocusing gel electrophoresis. J. Gen. Virol. 73, 1025-1029 (1992)).

Prusiner, and his colleagues were the first to purify $PrP^{Se}$ and demonstrate physical linkage to scrapie infectivity (Bolton et al., Identification of a protein thath purifies with the scrapie prion. Science 218, 1309-1311 (1982)). A collaboration between the groups of Prusiner, Hood and Weissmann led to the isolation of PrP cDNA and to the realization that $PrP^C$ was a normal host protein and that $PrP^{Se}$ was an isoform of $PrP^C$ (Oesch et al., vide supra (1985)). Weissmann and his collaborators (Basler et al., Scrapie and cellular PrP isoforms are encoded by the same chromosomal gene. Cell 46, 417-428 (1986)) cloned the PrP gene (*Prn-P*) and Prusiner's group showed the linkage between genetic susceptibility to prion disease and the Prn-p gene in mouse (Prusiner et al., vide supra (1990)) and man (Hsiao et al., Linkage of a prion protein missense variant to Gerstmann-Straussler syndrome. Nature 338, 342-345 (1989)). Several groups reported physical data supporting conformational differences between $PrP^C$ and $PrP^{Se}$ (Caughey et al., Secondary structure analysis of the scrapie-associated protein PrP 27-30 in water by infrared spectroscopy. Biochemistry 30, 7672-7680 (1991); Cohen et al., Structural clues to prion replication. Science 264, 530-531 (1994); Huang et al., Proposed three-dimensional structure for the cellular prion protein. Proc. Natl. Acad. Sci. U.S.A. 91, 7139-7143 (1994); Pan et al., Conversion of alpha-helices into beta-sheets features in the formation of the scrapie prion proteins. Proc. Natl. Acad. Sci. U.S.A. (1993); Safar et al., Conformational transitions, dissociation, and unfolding of scrapie amyloid (prion) protein. J. Biol. Chem. 268, 20276-20284 (1993)).

Since there is no reliable marker of transmissible spongiform encephalopathy infectivity, the kinetics of replication of the infectious agent cannot be studied specifically since the physical carriers of prions are not known. However, an increasing body of evidence from early experiments and from recent studies points to the importance of two distinct phases of replication during the life cycle of the prion, the infectious agent causing spongiform encephalopathies. In the first phase, replication of infectivity is thought to take place primarily in lymphoid organs (Eklund et al., Pathogenesis of scrapie virus infection in the mouse. J. infect. Dis. 117, 15-22 (1967); Clarke, M.C. and Komberlin, R.H. Pathogenesis of mouse scrapie: distribution of agent in the pulp and stroma of infected spleens. Vet. Microbiol. 9, 215-225 (1984); Fraser, H. and Dickinson, A.G. Studies of the lymphoreticular system in the pathogenesis of scrapie: the role of spleen and thymus. J. Comp. Pathol. 88, 563-573 (1978)). For example, infectivity can be demonstrated in the spleen as early as 4 days after i.p. or i.c. infection. This is true even if infection takes place via the intracerebral route (Kimberlin, R.H. and Walker, C.A. Pathogenesis of experminetal scrapie. Ciba. Found. Symp. 135, 37-62 (1988)), and replication of the infectious agent in the spleen precedes intracerebral replication even if infectivity is administered intracerebrally (Rubenstein et al., Scrapie-infected spleen: analysis of infectivity, scrapie-associated fibrils, and protease-resistant proteins. J. infect. Dis. 164, 29-35 (1991)). Infectivity can also accumulate in other components of the lymphoreticular system (LRS), e.g. in lymph nodes and in Peyer's plaques of the small intestine, where replication of infectivity can be demonstrated almost immediately following oral administration of prion preparations. The rapid establishment of a plateau of the infectious titer in the spleen at a relatively early time point during the latency time suggests that the availability of prion repication sites is rate-limiting in the LRS. It is not known however, whether this plateau is due to a limited number of spleen cells supporting prion replication, or rather to limited availability of prion replication sites within each cell. The nature of the cells supporting prion replication within the LRS is uncertain. Indirect evidence obtained by studies in which the spleen was removed at variable intervals after i.p. infection suggests that.the critical tissue compartment is long-lived and does not consist primarily of lymphocytes. In addition, ablation of lymphocytes by total body irradiation does not seem to affect the incubation time of mouse scrapie (Fraser et al., The scrapie disease process is unaffected by ionising radiation. Prog. Clin. Biol. Res. 317, 653-658 (1989)). Taken together, these and other findings suggest that follicular dendritic cells (FDC) may be the main population of cells involved in LRS replication of prions. Indeed, PrP accumulates in FDCs in the spleen of wild-type and nude mice, and i.p. infection does not lead to cerebral scrapie in SCID mice (whose FDCs are thought ot be functionally impaired) while it efficiently provokes the disease in nude mice which bear a selective T-cell defect (Muramoto et al., Species barrier prevents an abnormal isoform of prion protein from accumulating in follicular dendritic cells of mice with Creutzfeldt-Jakob disease. J. Virol. 67, 6808-6810 (1993)).

Though above delineated steps are thought to be important in the natural history of transmissible spongiform encephalopathy within an infected organism, the limiting factor or physical entity involved in the development and spread of transmissible spongiform encephalopathy after peripheral infection, that is to say the physical carrier of the prion, is still not known. Even though precise monitoring of the epidemic spread of transmissible spongiform encephalopathy is rendered extremely difficult by the long incubation times involved (up to 30 years), it appears to be likely that peripheral infection, e.g. by alimentary exposure, is the most relevant route of propagation. Any attempt to combate transmissible spongiform encephalopathy should thus focus on such limiting factors or physical entities involved in the development of the disease after periferal infection.

Knowledge about the identity of the physical carriers of prions would allow the design of improved assay methods for determining the infectivity of potentially infective materials like blood products or tissue derived products and for an improved monitoring of the epidemic progress of transmissible spongiform encephalopathy within infected populations. Also, knowledge about the interaction of the physical carriers of prions with further physical entities involved in pathogenesis would allow the monitoring of the disease progress within an infected victim.

Accordingly, there is an urgent need for the specific identification of the limiting factors and physical entities in the development of spongiform encephalopathy after peripheral infection.

Further, there is a need in providing improved assay methods for the diagnosis and/or monitoring of the progress or regress of transmissible spongiform encephalopathy in infected organisms or in organisms suspected of being infected. Such assay methods are also needed for the safety testing of body fluid or tissue derived products derived from such organisms.

Satisfaction of above needs as well as of further needs which will become apparent hereinafter is an object of the present invention.

## Summary of the invention

In order to meet above objects and to satisfy above needs, in one embodiment, the present invention provides an assay method for determination of the presence of transmissible spongiform encephalopathy in body fluid or tissue derived products isolated from human or animals, characterized in that said method comprises the steps of collecting the B-cells and/or the T-cells from a test sample and testing one or both cell types for the presence of transmissible spongiform encephalopathy by inoculating said cells into the cerebrum of a non-human test animal, wherein the development of transmissible spongiform encephalopathy in said test animal indicates the presence of said pathology in the body fluid or tissue derived products from which the tested cells were extracted.

In a further embodiment, the present invention provides an assay method for determination of the presence of infective T-cells in humans or animals or in body fluid or tissue derived products isolated therefrom.

In a further embodiment, the present invention provides an assay method for the monitoring of the progress of transmissible spongiform encephalopathy in humans or animals.

Further embodiments of the present invention are set out in the dependent claims.

## Detailed description of the invention

The present invention involves detailed investigations about' the nature of the limiting factors and/or physical entities in the development of spongiform encelophalopathy after periferal infection. Thus, the present invention involves identification of the physical carriers of prions and of the mechanisms involved in the spread of infectivity.

### Definitions

As referred to in the present application, B-cells (or B-lymphocytes) are to be understood as members of a subset of lymphocytic cells which are precursors of plasma cells which produce antibodies; they are able to recognize free antigens and antigens located on cells.

As referred to in the present application, T-cells (or T-lymphocytes) are to be understood as members of a subset of lymphocytic cells responsible for cellular immunity and the production of immunomodulating substances.

As referred to in the present application, the term 'lymphocytes' designates cells which participate in the humoral and cell-mediated immune defense, and which accordingly comprise B-cells and T-cells.

As referred to in the present application, the term 'animals' encompasses all eukaryotic organisms excluding plants.

### Figures

**Figure 1** shows the brain histopathology of immune deficient and control mice after i.p. inoculation of scrapie prions. The hippocampal formation was immunostained for glial fibrillary acidic protein, and identical segments of the pyramidal cell ribbon were microphotgraphed (200x). Intense, diffuse gliosis was visible in brains of T-cell-deficient, SCID, TNF-r$1^{0/0}$, *t11 μMT*, and infected control mice. Some *rag*-$2^{0/0}$ and μMT mice showed spongiform encephalopathy, but others of the same genotype did not display any pathology after similar time periods following i.p. inoculation, and were indistinguishable from mock-infected C57BL/6 mice.

**Figure 2** relates to the Western blot analysis of brains of immune-deficient mice after i.p. inoculation with transmissible spongiform encephalopathy prions and lack of specific antibodies against PrP in t11μMT mice. **Figs. a,b** are Western blots of brain material electrophoresed native (-) or after digestion with proteinase K (PK) (+). Large amounts of PK-resistant prion protein ($PrP^{SC}$) were detected in all mice that had developed spongiform encephalopathy, as well as in one *agr*$^{0/0}$ (**a**) two *rag-2*$^{0/0}$ and two μMT mice (**b**). One further B-cell-deficient mouse proved negative for $PrP^{SC}$ (not shown), and no clinical symptoms of spongiform encephalopathy were detected in any B-cell-deficient mice irrespective of accumulation of $PrP^{SC}$. **Fig. c** shows a Western blot prepared with recombinant murine PrP from *E. coli* ($PrP^R$), total brain protein extract from a wild-type mouse (WT), and total brain protein extract from a *Prnp*$^{0/0}$ mouse (0/0)[15]. Blots were incubated with serum from a t11μMT mouse inoculated with prions i.p. (left), stripped and reprobed with monoclonal antibody 6H4 to recombinant PrP (right). The presence of PrP-specific antibodies, as indicated by a 20K band in lane $PrP^R$ and by a cluster of bands present in lane WT but absent from lane 0/0, is evidence with 6H4 antibody but undetectable in t11μMT serum. Relative molecular mass markers (top to bottom): 105K, 82K, 45K, 37.3K, 28.6K, 19.4K. **Fig. d** shows the FACS analysis of immunoreactivity of *t11μMT* serum. Ordinate: cell counts; abscissa: logarithm of fluorescence intensity. Serum from a t11μMT mouse 210 days after i.p. inoculation with prions was diluted 1:10 and 1:100, stained VSV-infected EL4 cells (top panel, unfilled area) almost as strongly as VSV-specific monoclonal antibody VI24 (filed area). In contrast, immunoreaction of *t11μMT* serum (1:10) with $CD3^+$ T-cells from C57BL/6, *tga20, tg33* (ref.29) and $Prnp^{0/0}$ mice (lower panels) did not exceed background, like normal C57BL/6 serum on EL4 cells (top panel, dotted line). The same profiles were obtained when probes were stained with serum of untreated *t11μMT* mice (data not shown).

**Figure3a an 3b** display a flow analysis printout showing enriched B-cell and T-cell populations.

**Figure 4** Shows the infectivity of splenocytes in Wild type mice and Spleen mice.

**Figures 5a-5c** Show the infectivity in different cell types of Wild type mice, T-cell mice and Spleen mice.

## Investigations carried out by the inventors

### The role of the B-cells

As apparent from the prior art, the development of neurological disease after peripheral infection with transmissible spongiform encephalopathy depends on abnormal prion expansion within the cells of the lymphoreticular system [3,4]. The skilled man is however aware that the immune system comprises several components whose identity and precise function and specific interaction with the remaining components are still the object of extensive scientific investigation. The inventors have investigated for the first time the roles of different components of the immune system by using a panel of immune-deficent mice inoculated with prions intraperitoneally and found that defects effecting only T-cells had no apparent effect, but that all mutations that disrupted the differentiation and response of B-cells prevented the development of clinical spongiform encephalopathy. As an absence of B-cells and of antibodies correlates with severe defects in follicular dendritic cells, a lack of any of these three components may prevent the development of clinical spongiform encephalopathy. The key function of the follicular dendritic cells has been postulated inter alia by *Muramoto,* vide supra. However, the inventors found surprisingly that spongiform encephalopathy developed after peripheral inoculation in mice expressing immunoglobulins that were exclusively of the M subclass and without detectable specificity for the normal form of the prion $PrP^C$, and in mice which had B-cells but no functional follicular dendritic cells. Thus, the inventors have found out that differentiated B-cells are crucial for neuroinvasion by spongiform encephalopathy, regardless of the specificity of their receptors.

The effect of combined immune defects on the pathogenesis of spongiform encephalopathy was studied in mice deficient in *rag-2* (ref.5) and *rag-1* (ref.6), which lack B- and T-cells, in *scid* (severe combined immune deficient) mice, and in *agr*$^{0/0}$ mice, which lack rag-2 as well as the receptors for interferon-α/β[7] and interferon-γ[8]. Such mice were obtained according to methods well-known in the art of genetic engineering. For controls, inbred mice of strains C57BL/6 and 129/Sv which are the genetic backgrounds of all other mouse strains used were inoculated as well. To investigate the role of T-cells, the inventors used mice with targeted disruption of the genes encoding CD4 (ref. 9), CD8 (ref. 10), $\beta_2$-microglobulin[11] or perforin[12]. Selective depletion of B-cells was studied in μMT mice[13], which have a targeted disruption of the transmembrane exon of the immunoglobulin u-chain gene, do not produce any immunoglobulins and suffer from a B-cell differentiation block at the large-to small pre-B-cell transition, yet bear complete and functional T-cell subsets.

After intracerebral (i.c.) challenge with prions, all immune-deficient mice developed clinical symptoms of spongiform encephalopathy. This was confirmed by histopathological analysis (not shown) and by transmission of disease to indicator tga20 mice, which over-express the normal prion protein ($PrP^C$) and are hypersensitive to spongiform encephalopathy[14] (Table 1). Transmission to $Prnp^{0/0}$ mice[15], which do not express $PrP^C$ and are resistant to spongiform encephalopathy[16] (n=4), did not induce disease after >210 days, as expected for bona fide spongiform encephalopathy. In all groups, latency times from inoculation to first appearance of clinical symptoms and to terminal disease (Table 2), as well as brain prion infectivity titres (Table 1), were similar to those of control mice.

Thus, if prions where delivered to the central nervous system, spongiform encephalopathy pathogenesis and prion expansion in the brain proceeded without any detectable influence of the immune status of the host.

When mice were exposed to prisons through the intraperitoneal (i.p.) route, mice homozygous-null for CD4, CD8, $\beta_2$-microglobulin or perforin developed the initial symptoms of disease and terminal spongiform encephalopathy with latency periods similar to those of C57BL/6 and 129/Sv mice (Table 2), and reached analogous prion titres in both spleen and brain (Table 1). Thus the inventors concluded that $CD8^+$ cytotoxic and $CD4^+$ helper T-cells are not rate-limiting for spongiform encephalopathy after peripheral inoculation of prions, in agreement with the observation that nude mice develop spongiform encephalopathy normally after i.p. inoculation[3].

In contrast, no disease appeared after i.p. inoculation in μMT and in rag-deficient (*rag-1*$^{0/0}$, *rag-2*$^{0/0}$ and *agr*$^{0/0}$) mice, and no prion infectivity was detectable in their spleens (Table 1). In SCID C57BL/6 Mice, disease was marginally prolonged, which disagrees with earlier results[4,17] and may be due to incomplete immune deficiency of SCID mice in specific genetic backgrounds[18,19] , because SCID C.B-17 mice (whose immune defect is less leaky) did not develop disease (Table 2). B-cell differentiation to immune competence exhibits redundancy at many points; that renders such cells only partially sensitive to genetic manipulation.

Histopathological examination of brain sections revealed generalized spongiform encephalopathy in all wild-type and immune-deficient mice clinically diagnosed as spongiform encephalopathy-sick (Fig. 1). In addition, and despite lack of clinical symptoms, spongiform encephalopathy was seen in 1/7 rag -deficient and 1/6 μMT mice (at random sampling) 342 and 436 days after i.p. inoculation (Fig. 1), and significant prion titres were found in brains of 3/7 rag-deficient mice and 1/3 μMT mice (Table 1). Western blot analysis revealed accumulation of the disease form of prion, $PrP^{SC}$, in the brains of 2/6 rag -deficient and 2/6 μMT mice inoculated i.p. (Fig. 2). The remaining rag-deficient and μMT mice did not accumulate $PrP^{SC}$ as late as 504 days after inoculation.

For the sake of absolute scrutiny, it may be concluded that the latter findings are compatible with incipient spongiform encephalopathy in a minor fraction of B-cell-deficient mice. Therefore, although it prevents 'neuroinvasion' of the spongiform encephalopathy agent in most cases, absence of B-cells uncovers a slower, <50% efficient mechanism of pathogenesis which may cause spongiform encephalopathy in situations of immune deficiency. It should be emphazised that even then, B-cell deficiency prolongs the delay between $PrP^{SC}$ accumulation, onset of spongiform encephalopathy histopathology and clinical symptoms beyond the typical life expectancy of mice.

These results suggest that B-cells may 'transport' prions from lymphoid organs to nervous tissue. Alternatively, the apparent protection of B-cell-deficient mice from prions administered i.p. may result from the absence of immunoglobulins. Complexing of $PrP^{SC}$ with antibodies may favour nucleation (a process proposed to underlie the formation of prion infectivity[20]) or may opsonize $PrP^{SC}$ and enhance access to lymphoid sites of abnormal prion expansion. It also may suggest that animals become more able to propagate infection if the genetic change is later in B-cell development. To clarify this question, the inventors inoculated t11μMT mice (μMT mice expressing a rearranged IgM transgene directed against the glycoprotein of vesicular stromatitis virus) and found that they could support normal B-cell differentiation but exclusively expressed the transgenic IgM heavy chain, had a heavily skewed and very limited antibody repertoire, and lacked immunoglobulins of the D, G, E and A subclasses. Such mice were obtained according to methods well-known in the art.

After i.p. inoculation with prions, t11μMT mice developed disease with a latency comparable to that of wild-type mice (Table 2) and accumulated $PrP^{SC}$ in their brains (Fig. 2b). Serum from both uninfected and terminally spongiform encephalopathy-sick t11MT mice inoculated i.p. was shown by western blotting and by flow-assisted cell sorting (FACS) analysis not to crossreact with $PrP^C$ (Fig. 2c, d), suggesting that IgGs are not the effectors of prion 'neuroinvasion', and that a specific humoral immune response (at least as assessed by FACS and western-blot analysis) cannot be correlated with peripheral pathogenesis of spongiform encephalopathy. However, for the sake of absolute scrutiny, one cannot exclude the possibility that IgMs below the threshold of detectability, or indirect effects of antibodies, may be involved in spongiform encephalopathy pathogenesis. This corresponds to the difficulty in obtaining reliable disease transmission from soluble serum components from diseased animals.

B-cells are required for maturation of follicular dendritic cells (FDCs) and formation of germinal centres. Protection of B-cell-deficient mice may therefore result from the absence of FDCs, especially as FDs accumulate $PrP^{SC}$ extensively in i.p.-inoculated mice[3] and in the tonsils of patients suffering from new variant CJD[21]. Thus, the inventors inoculated mice lacking tumour-necrosis factor receptor-1 (TNF-R1$^{0/0}$)[22], which have virtually no germinal centres in lymphatic organs and very few, if any, FDCs[23], despite differentiation of functional B- and T-cells. These mice developed spongiform encephalopathy after both i.c. and i.p. inoculation, as did control mice (Table 2), thus disproving a prime role for FDCs in peripheral pathogenesis and supporting the inventors previous results that adoptive transfer of fetal liver cells (which does not efficiently replace FDCs[24]) can restore high spleen prion titres after i.p. inoculation[25].

TABLE 1

| Table 1 Scraple symptoms, scraple histopathology and infectivity titres in immunodeficient mice | | | | | |
|---|---|---|---|---|---|
| | | Primary infection (route of Inoculation: I.c.) | | Infectivity bioassay | |
| Genotype | Incubation (d) | Symptoms | Pathology | Brain | Spleen |
| CD-$4^{0/0}$ | 176 | + | + | 7.2*(65d ± 2†) | 6.6(71d±2) |
| CD-$4^{0/0}$ | 154 | + | + | 7.4(63d±1) | 7(67d±2) |
| *scid* | 167 | + | + | 7.3(64d±1) | 5.5(81d±2) |
| *scid* | 167 | + | + | 7.6 (62d±2) | 5.2 (83d±1) |
| *rag-2*$^{0/0}$ | 171 | + | + | 73(64.5d±2) | <0(>200d) |
| *agr*$^{0/0}$ | 182 | + | + | 7.3(64.5d±1) | <0(>145d) |
| *μMT* | 175 | + | + | 7.9(59d±5) | <0 (>200d) |
| | | Primary infection (route of Inoculation: I.p.) | | Infectivity bioassay | |
| CD.$4^{0/0}$ | 191 | + | + | 7.3(64d±1t) | ND |
| CD-$4^{0/0}$ | 195 | + | + | 7.5(62.5d±2) | ND |
| *scid* | 214 | + | + | 73(64d±1) | 5.2(83d±1) |
| *scid* | 249 | + | + | 7.7(61d±2) | 5(85d±1) |
| *rag-2*$^{0/0}$ | 286 | - | + | 6.5(72d±2) | <0(>200 d) |
| *rag-2*$^{0/0}$ | 286 | - | - | <0(>200d) | <0(>200 d) |
| *rag-2*$^{0/0}$ | 339 | - | - | <1 (>122d) | <2(>115 d) |
| *rag-2*$^{0/0}$ | 342 | - | + | 7.5(65d±1) | <2(>115 d) |
| *rag-1*$^{0/0}$ | 222 | - | - | <1 (>122d) | <2(>115 d) |
| *agr*$^{0}$ | 284 | - | + | 7.2(65d±0) | <0(>139 d) |
| *agr*$^{0/0}$ | 349 | - | - | <0(>139d) | <0(>139 d) |
| *μMT* | 286 | - | - | <0(>200d) | <0(>200 d) |
| *μMT* | 286 | - | - | <0(>200d) | <0(>200 d) |
| *μMT* | 375 | - | - | 7.8(60d±1) | <0(>200 d) |
| *μMT* | 436 | - | + | ND | ND |
| TNF-R$1^{0/0}$ | 211 | + | + | 7.7(61d±1) | ND |
| TNF-R$1^{0/0}$ | 212 | + | + | 7.7(60d±1) | ND |

For infectivity bioassays, brain or spleen homogenates were injected intracerebrally into groups of four *tga20* mice.
ND. not determined.
* prion titres expressed as log $LD_{50}$ per g of spleen or brain tissue.
† Incubation time, in days, of indicator *tga20* mice (average ± standard deviation).

| Table 2 Latency of scrapie in different immunodeficient mice | | | | | |
|---|---|---|---|---|---|
| | | Intracerebral route | | Intraperitoneal route | |
| Defect | Genotype | Scrapie | Time to terminal disease (d) | Scrapie | Time to terminal disease (d) |
| T | CD-$4^{0/0}$* | 7/7 | 159±11 | 8/8 | 191±1 |
| T | CD-$8^{0/0}$* | 6/6 | 157±15 | 6/6 | 202±5 |
| T | $\beta_2 \cdot \mu^{0/0}$* | 8/8 | 162±11 | 7/7 | 211±6 |
| T | *Pertorin*$^{0/0}$* | 3/4t | 171±2 | 4/4 | 204±3 |
| T and B | SCID* | 7/8t | 160±11 | 6/8‡ | 226 ± 15 |
| T and B | SCID§ | 4/4 | 152±1 | 1/4ll | 289 |
| T and B | *rag-2*$^{0/0}$* | 7/7 | 167±2 | 0/7 | Healthy (>504) |
| T and B | *rag-1*$^{0/0}$* | 3/3 | 175 ± 2 | 0/5 | Healthy (>258) |
| T and B | *agr*$^{0/0}$§ | 6/6 | 184±10 | 0/7 | Healthy (>450) |

(continued)

| Table 2 Latency of scrapie in different immunodeficient mice | | | | | |
|---|---|---|---|---|---|
| | | Intracerebral route | | Intraperitoneal route | |
| Defect | Genotype | Scrapie | Time to terminal disease (d) | Scrapie | Time to terminal disease (d) |
| B | μMT* | 8/8 | 181±6 | 0/8 | Healthy (>534) |
| IgG | *t11μMT** | 5/5 | 170±3 | 4/4 | 223±2 |
| FDC | TNF-R1$^{0/0}$# | 7/7 | 165±3 | 9/9 | 216±4 |
| Controls | 129Sv | 4/4 | 167±9 | 4/4 | 193±3 |
| | C57BL/6 | 4/4 | 166±2 | 4/4 | 206±2 |

All mice developed spongiform encephalopathy after I.c. inoculation. In contrast. B-cell-deficient mice stayed healthy after i.p. inoculation of RML scrapie prions.
* Genetic background was Inbred C57BL/6.
† One *Perforin$^{0/0}$* and one SCID mouse suffered from intercurrent death 136 and 141 days after inoculation, respectively.
‡ Two SCID C57BL/6 mice remained healthy and were killed 303 and 323 days after inoculation.
§ Genetic background was inbred C.B-17.
ll13/4 SCID C.B-17 mice remained healthy (>340d). Four further SCID C.B-17 mice were challenged with 100μl of a $10^{-1}$ dilution of RML prions, and all remained healthy (>340d).
∫ Genetic background was C57BL/6 x 129Sv.
# Genetic background was inbred 129Sv

Thus, the inventors have identified B-cells and B-cell-dependent processes as a limiting factor in the development of transmissible spongiform encephalopathy after peripheral infection. Accordingly, the present invention provides a novel, specific and therefore more preferable procedure to suppress that component of the immune system which is responsible for the prion spread, namely the B-cells.

<u>The role of the T-cells</u>

Still further, the inventors have studied the role of B-cell-dependent processes during pathogenesis. Accordingly, the inventors have carried out further experiments aiming at establishing the amount and nature of possible interaction of infective B-cells with the remaining components of the immune system, e.g. with T-cells. Results of the inquiry about such interaction and design of suitable therapeutic measures influencing such interaction are a further aspect influencing the present invention.

As pointed out above, it is known that mice devoid of functional PrP genes (Prn-p$^{0/0}$) are resistant to transmissible spongiform encephalopathy and do not propagate prions (Büeler et al. Cell, 73, 1339-1347, 1993). Thus, reintroduction of PrP transgenes into Prn-p$^{0/0}$ should restore transmissible spongiform encephalopathy. Departing from this concept, the inventors conducted studies in Prn-p$^{0/0}$ mice transgenic for PrP genes controlled by tissue specific promotors. Such mice may be obtained by the man skilled in genetic engineering according to methods well-known in the art. Specifically, the inventors used 'T-cell mice' (Ick promotor; Chaffin et al., EMBO J. 9, 3821-3829) which express PrP exclusively in T-cells and 'spleen mice' (IRF-1 promoter/Eu enhancer; Yamada et al., Proc.Natl.Acad.Sci.USA. 88, 532-536, 1991) which express PrP in splenocytes and at low level in brain. Challenge of spleen-mice with prions led to the development of spongiform encephalopathy in that spleen mice succumbed at a late stage due to brain disease and showed propagation of prions in spleen and thymus as well as in brain. On the other hand, T-cell mice showed no propagation of prions. Accordingly, these results are fully consistent with the prior experiments as described hereinabove and confirm the crucial role of B-cells (Table 3).

TABLE 3

| *Mice* | *Incubation time Mean Days ± sd* | *n/no* |
|---|---|---|
| Prn-p$^{+/+}$ | 196±4 | 10/10 |
| Prn-p$^{0/0}$ | >500 | 0/3 |
| "Spleen mice" | 263±5 | 7/13 |
| "T cell mice" | >500 | 0/5 |

Table 1: Transmission of mouse prions to transgenic mice with ectopic PrP expression

To further investigate the role of B-cell dependent processes, in a second step, the infectivity of the splenocytes from spleen mice was selectively determined in a bioassay. It was found that, though most of the infectivity was indeed carried by the B-cells, the T-cell-s were also contaminated to some extent (Table 4).

TABLE 4

| *Cell Fraction* | Titer *(LD50 units/$10^8$ cells)* |
|---|---|
| splenocytes | -200 |
| B cells | -500 |
| T cells | -100 |
| non-B, non-T cells | <1 |

Table 2: Infectivity of total and fractionated splenocytes from "Spleen mice" 120 days after i.p. inoculation with prions. cells were fractionated by magnetic activated cell sorting (MACS) using anti-B220 antibodies for B cells and anti-Thy 1.2 antibodies for T-cells.

This newly found contamination shown by the T-cells of the spleen mice seems to be in contradiction with the fact that the T-cells of T-cell mice do not show any contamination (see Figures 4 and 5). However, what initially seems to be a contradiction (infectivity of T-cells in some cases, non-infectivity of T-cells in other cases), in reality implies and supports the existence of an interaction between the B-cells (the carriers of infectivity and the T-cClls (which, as such, are not able to propagate infectivity). Spleen mice contain both T-cells and B-cells, and upon infection of the B-cells, a B-cell mediated secondary infection of the spleen mice T-cells takes place. On the contrary, T-cell mice do not contain B-cells, and as a consequence of this lack of infectivity carriers, the T-cell mice T-cells are not subject to infection.

Conclusions

As pointed out above, not only the crucial carrier of infectivity, namely the B-cells, has been identified, but also a powerful tool for the monitoring of the spread of transmissible spongiform encephalopathy within the immune system of an infected human or animal has been provided for the first time by the inventors. Indeed, the present invention allows the distinction between the occurence of infected B-cells alone and the further occurence of secondarily infected T-cells.

Further aspects and preferred embodiments of the invention

A further object of the present invention is the provision of a diagnostic method allowing the determination of the presence of transmissible spongiform encephalopathy in body fluid or tissue derived products isolated from humans or animals and comprising the steps of collecting B-cells from body fluids or from tissue or from products derived therefrom and inoculating said B-cells into the cerebrum of a non-human test animal, development of transmissible spongiform encephalopathy in said test animal indicating presence of said pathology in the body fluids or tissue derived products from which the B-cells were extracted.

As to the collection step, the invention contemplates any method known in the art suitable for selective collection of B-cells or of their progenitors or products from said body fluid or tissue sample.

As will be apparent to the man skilled in the art, such collection involves use of a reactive physical entity specifically recognizing B-cells, preferably B-cell specific antibodies, as the ones described hereinbelow.

In a preferred but not limiting embodiment of the present invention, the inventors have used anti-mouse-B220 antibodies conjugated with super-paramagnetic microbeads (Milteny Biotec GmbH, Germany) for the purification of B-cells.

Suitable non-human test animals for carrying out the method of the present invention are e.g. tga20 indicator mice and as they were used by Brandner et al. in 'Normal host prion protein necessary for scrapie-induced neurotoxicity', Nature, 379, (1996), the disclosure of which is hereby incorporated by reference. As reported by Brandner, infectivity of a given inoculum determines the incubation time elapsed before the appearence of clinical symptoms displayed by the test animals. (see Table 5) Accordingly, the use of purified fractions containing high titers of B-cells constitutes an advantage provided by the assay methods of the present invention.

Though any suitable method known to the man skilled in the art could be used for the specific collection of B-cells from body fluids, specific collection by means of B-cell specific immunoreactants like e.g. B-cell specific antibodies is preferred. Suitable but not limiting examples of such B-cell specific antibodies are commercially available B220 or LR1 antibodies or anti-μM antibodies, vide supra. The term "specific collection by means of B-cell specific antibodies" encompasses any collection method which comprises the use of collection reagents comprising B-cell specific antibodies for the recognition of B-cells in body fluid products. Collection reagents comprising B-cell specific antibodies are B-cell specific antibodies which are conjugated to a solid phase or which are capable of interacting with a solid phase via chemical or physical means either by themselves or by virtue of suitable derivatization in such a manner that they get either directly or indirectly immobilized on said solid phase so as to enable collection from the reaction mixture.

Though any suitable method known to the man skilled in the art could be used for the specific collection of B-cells from tissue derived products, specific collection by means of B-cell specific immunoreactants like e.g. B-cell specific antibodies is preferred. Suitable but not limiting examples of such B-cell specific antibodies are commercially available B220 or LR1 antibodies or anti-μM antibodies. The term "specific collection by means of B-cell specific antibodies" encompasses any collection method which comprises the use of collection reagents comprising B-cell specific antibodies for the recognition of B-cells in tissue derived products. Collection reagents comprising B-cell specific antibodies are B-cell specific antibodies which are conjugated to a solid phase or which are capable of interacting with a solid phase via chemical or physical means either by themselves or by virtue of suitable derivatization in such a manner that they get either directly or indirectly immobilized on said solid phase so as to enable collection from the reaction mixture.

A further aspect of the present invention relates to the B-cell mediated secondary infection of T-cells.

In view of the above, the present invention provides a diagnostic method allowing the determination of the presence of transmissible spongiform encephalopathy in body fluid or tissue derived products isolated from humans or animals and comprising the steps of collecting T-cells from body fluids or from tissue or from products derived therefrom and inoculating said T-cells into the cerebrum of a non-human test animal, development of transmissible spongiform encephalopathy in said test animal indicating presence of said pathology in the body fluids or tissue derived products from which the T-cells were extracted.

As to the collection step, the invention contemplates any method known in the art suitable for selective collection of T-cells or of their progenitors or products from a body fluid or tissue sample drawn from said human or animal. As will be apparent to the man skilled in the art, such collection involves use of a reactive physical entity specifically recognizing T-cells, preferably T-cells specific antibodies, as the ones described hereinbelow. In a preferred but not limiting embodiment of the present invention, the inventors have used anti-mouse-Thyl.2 antibodies conjugated with super-paramagnetic microbeads (Milteny Biotec GmbH, Germany) for the purification of T-cells.

Though any suitable method known to the man skilled in the art could be used for the specific collection of T-cells' from body fluids, specific collection by means of T-cell specific immunoreactants like e.g. T-cell specific antibodies is preferred. A suitable but not limiting example of such a T-cell specific antibody is Thy1.2. The term "specific collection by means of T-cell specific antibodies" encompasses any collection method which comprises the use of collection reagents comprising T-cell specific antibodies for the recognition of T-cells in body fluid products. Collection reagents comprising T-cell specific antibodies are T-cell specific antibodies which are conjugated to a solid phase or which are capable of interacting with a solid phase via chemical or physical means either by themselves or by virtue of suitable derivatization in such a manner that they get either directly or indirectly immobilized on said solid phase so as to enable collection from the reaction mixture.

Though any suitable method known to the man skilled in the art could be used for the specific collection of T-cells from tissue derived products, specific collection by means of T-cell specific immunoreactants like e.g. T-cell specific antibodies is preferred. A suitable but not limiting examples of such a T-cell specific antibody is Thy1.2. The term "specific collection by means of T-cell specific antibodies" encompasses any collection method which comprises the use of collection reagents comprising T-cell specific antibodies for the recognition of T-cells in tissue derived products. Collection reagents comprising T-cell specific antibodies are T-cell specific antibodies which are conjugated to a solid phase or which are capable of interacting with a solid phase via chemical or physical means either by themselves or by virtue of suitable derivatization in such a manner that they get either directly or indirectly immobilized on said solid phase so as to enable collection from the reaction mixture.

As pointed out above, the present invention provides further an assay method for monitoring the progress of transmissible spongiform encephalopathy. Said assay method comprises the extraction of B-cells and T-cells from body fluid or tissue samples drawn from the human or animal undergoing diagnosis and testing for infection both cells by inoculating said cells in the cerebrum of a non-human test animal. In a further embodiment of this method, the B-cells are tested first for infection and, if they are found to be infected, then the T-cells are tested for infection.

Extraction of both physical entities can be carried out either simultaneously or sequentially. The purified B- and T-cell fractions thus obtained may be further purified by complement lysis of B-cells in the T-cell fraction and vice versa. Suitable but not-limiting examples for antibodies suitable complement lysis in vitro are rat anti mouse LR1 antibody (clone LR6.2B6D6.C9, Serotec) and mouse anti mouse antibody Thy1.2 (clone F7D5, Serotec).

The present invention will now be described by way of examples, which are meant to illustrate the scope of the present invention.

## EXAMPLES

### Example 1

*Generation of t11 μMT mice.*

The V-gene segment of the immunoglobulin heavy chain of the immunoglobulin heavy chain of the B-cell hybridoma VI41 (ref. 27) secreting a VSV-neutralizing antibody was cloned into an expression vector encoding the mouse μ-chain of allotype a. Transgenic mice were generated and backcrossed to μMT mice. *t11 μMT* mice exclusively expressed the transgenic μ-chain of the allotype a; endogenous IgM of the allogtye b and immunoglobulins of other subclasses were not detected in their serum (not shown).

### Example 2

1. Scrapie inoculation

Mice were inoculated with a 1% homogenate of heat- and sarcosyl-treated brain prepared from mice infected with the Rocky Mountain laboratory (RML) scrapie strain. Thirty microliter were used for intra-cranial (i.c.) injection, whereas 100μl were administered by intra-peritoneal (i.p.) route. Mice were monitored every second day, and scrapie was diagnosed according to standard clinical criteria.

2. Western-blot analysis

Ten percent brain homogenates were prepared as described[16] and, where indicated, digested with 20μg/ml of proteinase K for 30 minutes at 37° C. Eighty μg of total protein were then electrophoresed through 12% SDS-polyacrylamide gel, transferred to nitrocellulose membranes, probed with monoclonal antibody 6H4 (Prionics AG, Zurich) or polyclonal antiserum IB3 (reference 26) against mouse PrP, and developed by enhanced chemiluminescence.

3. Detection of PrP antibodies

Brain Lysates from wild-type and *Prnp*$^{0/0}$ mice, as well as recombinant *E. coli* PrP, were electrophoresed through a 12,5% SDS-polyacrylamide gel and transferred to nitrocellulose membranes. Membranes were then incubated with serum from infected, terminally scrapie-sick mice (1:100 diluted). Visualization was achieved by enhanced chemiluminescence as previously described for the Western-blot.

4. Immunohistochemical studies

Brain tissues from each mouse was fixed, inactivated for 1 hour with 98% formic acid, embedded in paraffin and subjected to conventional staining and to immuno-staining for glial fibrillary acidic protein according to standard procedure. Glioais (a nonspecific but early indicator of brain damage) was detected by the presence of large immunostained reactive astrocytes. In terminally scrapie-sick mice, wide spread vacuolation was consistently seen throughout the central vervous system.

5. Infectivity bioassays

Brain and spleen homogenate (w/v, 10% in 0,32 M sucrose) were prepared from infected animals as described, and 30μl (diluted 1:10 in phosphate buffered saline containing 1% BSA) were administered i.c. to groups of at least 4 *tga*[20] mice for each sample. The incubation time until development of terminal scrapie sickness was determined and infectivity titer were calculated using the realtionship $y = 14.37 - 0{,}11x$ where $y$ is the IDso and x is the incubation time (in days) to terminal disease.

6. Preparation of solenocytes

Spleens were recovered from mice at 34 days following i.p. inoculation with the RML strain of prions. Splenocyte suspensions were prepared by forcing spleens, through a fine mesh screen into 25ml of magnetic activated cell separation (MACS) buffer. The MACS buffer is composed of phosphate buffered saline containing 1% BSA, 5mM EDTA and 0,1% sodium azide. Following a 15 minute incubation on ice to allow the cell clumps to settle the cell suspension was removed for further evaluation.

7. Antibodies

Antibodies conjugated to super-paramagnetic microbeads which specifically recognize B and T cells (anti-mouse-B220, anti-Thy 1,2, anti-IgM, and anti-CD3) were obtained from Milteny Biotech GmbH. All magnetic separation columns (A2 & CS Column) were also obtained from Milteny Biotech GmbH. Rabbit complement was obtained from Cedarlane, Ontario (Low-tox-M rabbit complement). Additional antibodies (LR1, mouse anti-mouse thy 1.2) were obtained from Serotec.

8. B and T cell purification by magnetic bead separation

Five ml of a Splenocyte suspension was centrifuged at 100 rpm for 10 minutes and the cell pellet was recovered in = 0,6ml of MACS buffer. The cells were then incubated with 75μl of B-200 or Lthy 1,2 conjugated super-paramagnetic micrbeads as per manufacturer instruction (Milteny Biotech GmbH) for 15 minutes at 4° C. Following the incubation, the total volume was adjusted to 2ml with MACS buffer and loaded onto a prefilled and washed A2 column (magnetic separation column). Cells not associated with the magnetic microbeads were eluted with 5ml of MACS buffer. The column was then removed from the magnetic field and back flushed to remove the extracted cells. The separation process is then repeated and the final B or T enriched cell population is eluted with 11ml of MACS buffer after the separation column was removed from the magnetic field.

9. Complement lysis

To further improve the purity of the B and T cell population. abtained by magnetic separation, complement lysis of the T or B cell enriched population was performed. Cells were pelleted and resuspended in cytotoxicity medium (CM, RPMI-1640 media containing 25mM HEPES and 0,3% BSA) to a concentration of 1-3x$10^7$ cells/ml. For B cell depletion, a B cell specific antibody, e.g., LR1 was used. Whereas for TL cell depletion, a T cell specific antibody, e.g., Thy 1,2 was used. Optimal effective antibody concentration would need to be individually determined for the specific antibody sources. Incubation with the antibodies is performed at 4° C for 60 minutes after which the cells were resuspended in LCM containing 20% Low-tox-M rabbit complement and incubated at 37° C for 60 minutes to allow for cell lysis. Viable cells were then separated from the dead cells and debris by centrifugation over lympholyte-M (Cedarlane, Ontario) or other cell separation medium as according to the manufacturer instruction.

10. Cell preparation for Flow Cytometry analysis

Single cell suspension for flow cytometry analysis were prepared in FACS buffer consists of phosphate buffered saline containing 2% FCS, 20mM EDTA and 1% sodium azide. When peripheral blood samples were used, the lymphocyte population was enriched by lysis removal of the red blood cells from heparinized blood. The cell staining process consists of incubating cell population with saturating concentration of fluorescein (FITC)-conjugated antibodies for 30 minutes at 4° C. The cells were then washed with FACS buffer to remove the unbounded material and subject to flow analysis. When the indirect staining method was used, the cell populations were first incubated with the primary antibody for 30 minutes at 4° C, washed with FACS buffer and followed with an additional 30 minutes of incubation at 4°C with a secondary FITC-conjugated antibody. After removal of the unbounded FITC-conjugated secondary antibodies, the cell populations were then ready for flow analysis.

Discussion of the Results of example 2

1. Determination of scrapie infectivity

Infectivity of brain material from scrapie infected mice was demonstrated by i.c. infection of tga20 indicator mice. Infectivity was determined by injecting 30μl samples i.c. into tga20 mice and determining time to disease manifestation by standard histochemical procedure. Table 5 illustrates a typical outcome of such analysis. This analysis gives the success rate of disease transmission and the duration/incubation time for the expression of the disease symptoms. Hence the assay reveal the susceptibility of the host strain to the disease and, thus allow for the determination of the critical cell types necessary for disease transmission.

TABLE 5

| TABLE 5 Determination of scraple infectivity | | | |
|---|---|---|---|
| Source of Infectivity | Days after Inoculation | Transmission* | Incubation time of recipient (days) |
| (a) Standard prion Inoculum† | | | |
| RML, $10^{-1}$ | - | 4/4 | 59, 60, 63, 68 |
| RML, $10^{-3}$ | - | 2/2 | 66, 67 |
| RML, $10^{-5}$ | - | 4/4 | 84, 85, 85, 96 |
| RML, $10^{-7}$ | - | 1/3 | 109, >217, >217 |
| RML, $10^{-9}$ | - | 0/4 | >217, >217, >217, >217 |
| RML, $10^{-11}$ | - | 0/3 | >217, >217, >217 |

2. Evaluation of the potential target cells for scrapie transmission by genetic methodology

The effect of immune defects on the pathogenesis of scrapie was studied in mice deficient in T cell, B cell or with combined T/B cell defects. A number of different mouse genotypes that are suitable have been generated and the selection of the type to be used will be apparent to a person skilled in the art. The success of infection is determined by examination of the disease symptoms, pathology and by infectivity bioassay. Table 1 illustrate a typical outcome of such analysis. This analysis gives the incubation time- from infection to symptom presentation, the presence or absence of symptoms and pathological features. Further the infectivity bioassay provide information regarding the latency of the infective agents in the brain and splenic tissues of the primary infected host. By correlating the disease expression and genotype of infected animals, table 1 illustrates that if the infective agent is introduced by the i.c. route all genotypes express the disease regardless of their B cell or T cell defects. Alternatively, by examining the (secondary) infective capability of brain and splenic tissues from the primary infected hosts, the potential target cell lineage of scrapie transmission can be examined. Thus table 2 further illustrates that following i.c. inoculation, only those genotypes with intact B cell functions are capable of demonstrating secondary infectivity in the spleen tissues.

By taking a more peripheral route of primary infection, i.e., i.p. inoculation, the propagation of the disease can be further delineated. This is further illustrated in table 1. The analysis demonstrates that by selecting animals with specific lymphocyte defects, the critical lymphoid cell types for scrapie disease transmission can be specifically identified. These results suggest that B cells may "transport" prions from lymphoid organs to nervous tissues. (The mode of transport is not limited to direct cell associated transport but may also be complexes with various cellular products. The components is not limited to but may include antibodies, $PrP^{C}$, $PrP^{SC}$ and other similar cellular products).

3. Evaluation of the role of lymphoid cells in prion disease transmission

Cellular components of the peripheral lymphoid tissues, e.g., spleen, lymph nodes can be readily obtained from animals. Such conditions are described by public literature.

The cellular components obtained can be further separated by specific antibody to differential surface markers for the various lymphoid cell types which gas been conjugated to magnetic microbeads. By additional deletion of undesirable cell types by cytotoxic depletion using complement, highly purified cell isolates can be obtained. The procedure is constructed to isolate highly enriched T-cell and B-cell populations. The isolated cell populations are suspended in culture medium, e.g., RPMI-1640 and can be supplemented with serum and with additives like glutamic acid, growth factors, cytokines or other modulators of cell physiology prior to evaluation of infectivity capacity. Such highly enriched lymphocytes can be further characterized by Flow cytometry evaluation of the membrane surface components, e.g., CD-4, CD-8, and/or Ig expression and is obvious to a person skilled in the art. Figure 3a and 3b illustrate a typical Flow analysis of such enriched population. The cellular purity is demonstrated by the expression of T cell or B cell specific sufrace markers. Other non cell lineage associated components can also be documented by similar means, e.g., cell surface expression of $PrP^{C}$ and $PrP^{SC}$. Further, molecular biology techniques as described by public literature can also be employed to document non-membrane associated specific intracellular components, e.g., DNA, RNA, mRNA whose presence is indicative of ist cellular presence.

Such cellular lymphoid components can be obtained from infective and non-infective hosts and characterized for ist lineage and intracellular capacities. Subsequently, their infective capacity can be examined by inoculation via the i.c. or i.p. route. By this assay it is possible to determine the cell lineage most responsible for prion disease transmission. Further, by measurement of various intracellular components and correlation with the cellular lineage, the assay is indicative of the interactions between the prions and the tentative target cells.

TABLE 6

**Table 6 Infectivity in cell fractions of Tig94 and wt spleen**

| | | **Tg94 (1. Experiment)** | | **Tg94 (2. Experiment)** | | **wt** | |
|---|---|---|---|---|---|---|---|
| **Fraction** | **cells** | **Inc.time** | **n/no** | **Inc.time** | **n/no** | **Inc.time** | **n/no** |
| **splenocytes** | $10^6$ | 76±4 | (4/4) | nd | | nd | |
| | $10^5$ | 84.5±1 | (2/2) | 89±1 | (2/2) | 83.5±3 | (2/2) |
| | $10^4$ | 109±14 | (4/4) | 106.5±18 | (4/4) | 89±5 | (4/4) |
| | $10^3$ | 142 | (1/4) | 116.5±12 | (2/4) | 106±7 | (4/4) |
| | $10^2$ | 141±35 | (2/2) | >200 | (0/4) | >200 | (0/4) |
| **B cells** | **2x10$^5$** | **76±1** | (4/4) | nd | | nd | |
| | **2x10$^4$** | **87±1** | (3/4) | 106±10 | (4/4) | 88±2 | (4/4) |
| | **2x10$^3$** | **116±37** | (3/4) | 106±11 | (2/4) | 102±14 | (4/4) |
| | **2x10$^2$** | **110** | (1/4) | >200 | (0/4) | 91 | (1/4) |
| **T cells** | $10^5$ | 82±6 | (4/4) | nd | | nd | |
| | $10^4$ | 110±10 | (3/4) | 109.5±1 | (2/4) | 94±8 (4/4) | |
| | $10^3$ | 106 | (1/3) | >200 | (0/4) | 108±18 | (2/2) |
| | $10^2$ | 108 | (1/4) | >200 | (0/4) | >200 | (0/4) |
| **Non B/T cells** | **5x10$^5$** | **124.5±1** | (2/4) | 108 | (1/4) | 112±14 | (3/4) |
| | **5x10$^4$** | | (0/4) | nd | | >200 | (0/4) |

FACS analysis shown in Fig. 2D

Peripheral blood cells were incubated with serum from t11μMT mice, washed, incubated with anti-mouse IgM-FITC conjugate followed by anti-CD3-PE (Pharmingen), and analysed with a Becton-Dickinson FAScan instrument after erythrocyte lysis and fixation. For analysis, cells were gated on CD3-positive T-cells. EL4 cells infected with vesicular stomatitis virus (VSV) were stained with 5μg VSV-specific monoclonal antibody VI24 (ref.27) and with FTC-labelled antibody to mouse IgG2a (Southern Biotechnology), or with serum of t11μMT mice, and with FITC-labelled F(ab')2 antibody to mouse IgM (anti-IgM-FITC, Tago), or with serum of C57BL/6 mice and anti-IgM-FITC. All data acquisition and analysis were performed with CellQuest software (Becton Dickinson).

**Example 4 (comparative)**

Production of Antibodies Against Infective Lymphocytes

A. Production of Polyclonal Antisera.
Antiserum against infective lymphocytes (B-cells or T-cells) is prepared by injecting appropriate animals with infective lymphocytes identified and isolated as described in example 2.

1. Starting materials
Specifically, purified B-cell peparations and/or T-cell preparations are used. The whole cell preparations of infective lymphocytes can be used directly as immunogen or alternatively infective lymphocytes can be gently lysed with mild detergent treatment for example with 0.05-0.5% Triton X 100 followed by fixation in 0.5-2% paraformaldehyde in 1% PBS for 5-100 minutes at 4-10° C.
2. Animal Immunization. Female white New Zealand rabbits weighing 2 kg or more are used for raising polyclonal antiserum. Generally, one animal is immunized per infective lymphocyte preparation. One week prior to the first immunization, 5 to 10 ml of blood is obtained from the animal to serve as a non-immune prebleed sample.

Infective lymphocytes are used to prepare the primary immunogen by emulsifying 0.5 ml of the infective lymphocyte preparation at a concentration of between 1x10$^5$ to 1x10$^8$ cells/ml in PBS (pH 7.2) with 0.5 ml of complete Freund's adjuvant (CFA) (Difco, Detroit, MI). The immunogen is injected into several sites of the animal via subcutaneous, intraperitoneal, and/or intramuscular routes of administration. Four weeks following the primary immunization, a booster immunization is administered. The immunogen used for the booster immunization dose is prepared by emulsifying 0.5 ml of the same infective lymphocyte preparation used for the primary immunogen, except that 0.5 ml of incomplete Freund's adjuvant (IFA) (Difco, Detroit, MI) is now used. Again, the booster dose is administered into several sites and can utilize subcutaneous, intraperitoneal and intramuscular types of injections. The animal is bled (5 ml) two weeks after the booster immunization and the serum is tested for immunoreactivity to the infective lymphocyte preparation as described below. The booster and bleed schedule is repeated at 4 week intervals until an adequate titer is obtained. The titer or concentration of antiserum is determined by microtiter EIA as described in Example 17, below. An antibody titer of 1:500 or greater is considered an adequate titer for further use and study.

B. Production of Monoclonal Antibody.

1. Immunization Protocol . Mice are immunized using immunogens prepared as described hereinabove, except that the amount of the immunogen for monoclonal antibody production in mice is one-tenth the amount used to produce polyclonal antisera in rabbits. The primary immunogen consists of 0.1ml of the infective lymphocyte preparation at a concentration of between $1x10^5$ to $1x10^8$ cells/ml in PBS (pH 7.2) in 0.1 ml of CFA emulsion; while the immunogen used for booster immunizations consists of 0.1ml of the infective lymphocyte preparation as above emulsified with 0.1 ml of IFA. Hybridomas for the generation of monoclonal antibodies are prepared and screened using standard techniques. The methods used for monoclonal antibody development follow procedures known in the art such as those detailed in Kohler and Milstein, Nature 256:494 (1975) and reviewed in J.G.R. Hurrel, ed., Monoclonal Hybridoma Antibodies: Techniques and Applications, CRC Press, Inc., Boca Raton, FL (1982). Another method of monoclonal antibody development which is based on the Kohler and Milstein method is that of L.T. Mimms et al., Virology 176:604-619 (1990), which is incorporated herein by reference.

The immunization regimen (per mouse) consists of a primary immunization with additional booster immunizations. Booster immunizations are performed at approximately two weeks and four weeks post primary immunization. A total of 100 μl of immunogen is inoculated intraperitoneally and subcutaneously into each mouse. Individual mice are screened for immune response by microtiter plate enzyme immunoassay (EIA) as described in Example 17 approximately four weeks after the third immunization. Mice are inoculated either intravenously, intrasplenically or intraperitoneally with 0.1ml of the infective lymphocyte preparation at a concentration of between $1x10^5$ to $1x10^8$ cells/ml in PBS (pH 7.2) in 0.1 ml of IFA approximately fifteen weeks after the third immunization..

Three days after this intravenous boost, splenocytes are fused with, for example, Sp2/0-Ag14 myeloma cells (Milstein Laboratories, England) using the polyethylene glycol (PEG) method. The fusions are cultured in Iscove's Modified Dulbecco's Medium (IMDM) containing 10% fetal calf serum (FCS), plus 1% hypoxanthine, aminopterin and thymidine (HAT). Bulk cultures are screened by microtiter plate EIA following the protocol in Example 17. Clones reactive with the infectious lymphocyte preparation used as immunogen and non-reactive with non-infectious lymphocyte preparation (i.e., lymphocytes prepared from non-infected animals not used as the immunogen) are selected for final expansion. Clones thus selected are expanded, aliquoted and frozen in IMDM containing 10% FCS and 10% dimethyl-sulfoxide.

2. Production of Ascites Fluid Containing Monoclonal Antibodies. Frozen hybridoma cells prepared as described hereinabove are thawed and placed into expansion culture. Viable hybridoma cells are inoculated intraperitoneally into Pristane treated mice. Ascites fluid is removed from the mice, pooled, filtered through a 0.2 μ filter and subjected to an immunoglobulin class G (IgG) analysis to determine the volume of the Protein A column required for the purification.

3. Purification of Monoclonal Antibodies From Ascites Fluid. Briefly, filtered and thawed ascites fluid is mixed with an equal volume of Protein A sepharose binding buffer (1.5 M glycine, 3.0 M NaCl, pH 8.9) and refiltered through a 0.2 μ filter. The volume of the Protein A column is determined by the quantity of IgG present in the ascites fluid. The eluate then is dialyzed against PBS (pH 7.2) overnight at $2\text{-}8_i$C. The dialyzed monoclonal antibody is sterile filtered and dispensed in aliquots. The immunoreactivity of the purified monoclonal antibody is confirmed by determining its ability to specifically bind to the infectious lymphocyte preparation used as the immunogen by use of the EIA microtiter plate assay procedure of Example 17. The specificity of the purified monoclonal antibody is confirmed by determining its lack of binding to irrelevant non-infectious lymphocytes not used as the immunogen. The purified anti-infectious lymphocyte monoclonal thus prepared and characterized is placed at either $2\text{-}8_i$C for short term storage or at $-80_i$C for long term storage.

4. Further Characterization of Monoclonal Antibody. The isotype and subtype of the monoclonal antibody produced as described hereinabove can be determined using commercially available kits (available from Amersham. Inc., Arlington Heights, IL). Stability testing also can be performed on the monoclonal antibody by placing an aliquot of the monoclonal antibody in continuous storage at 2-8¡C and assaying optical density (OD) readings throughout the course of a given period of time.

C. Use of Recombinant Proteins as Immunogens. It is within the scope of the present invention that recombinant proteins made as described herein can be utilized as immunogens in the production of polyclonal and monoclonal antibodies, with corresponding changes in reagents and techniques known to those skilled in the art.

**Example 5 (comparative)**

Purification of Serum Antibodies Which Specifically Bind to Infectious Lymphocytes

Immune sera, obtained as described hereinabove in Example 4, is affinity purified using immobilized proteins from the infectious lymphocyte preparation used as the immunogen as described above. An IgG fraction of the antiserum is obtained by passing the diluted, crude antiserum over a Protein A column (Affi-Gel protein A, Bio-Rad, Hercules, CA). Elution with a buffer (Binding Buffer, supplied by the manufacturer) removes substantially all proteins that are not immunoglobulins. Elution with 0.1M buffered glycine (pH 3) gives an immunoglobulin preparation that is substantially free of albumin and other serum proteins.

Immunoaffinity chromatography is performed to obtain a preparation with a higher fraction of specific antigen-binding antibody. The infectious lymphocyte preparation used to raise the antiserum is immobilized on a chromatography resin, and the specific antibodies directed against its epitopes are adsorbed to the resin. After washing away non-binding components, the specific antibodies are eluted with 0.1 M' glycine buffer, pH 2.3. Antibody fractions are immediately neutralized with 1.0M Tris buffer (pH 8.0) to preserve immunoreactivity. A resin such as Affi-Gel 10 or Affi-Gel 15 is used (Bio-Rad, Hercules, CA). If coupling through a carboxy is desired, Affi-Gel 102 can be used (Bio-Rad, Hercules, CA). An organomercurial resin such as Affi-Gel 501 can be used (Bio-Rad, Hercules, CA).

Alternatively, spleens can be harvested and used in the production of hybridomas to produce monoclonal antibodies following routine methods known in the art as described hereinabove.

**Example 6 (comparative)**

Western Blotting of Tissue Samples

Protein extracts are prepared by homogenizing tissue samples in 0.1M Tris-HCl (pH 7.5), 15% (w/v) glycerol, 0.2mM EDTA, 1.0 mM 1,4-dithiothreitol, 10 μg/ml leupeptin and 1.0 mM phenylmethylsulfonylfluoride (Kain et al., Biotechniques, 17:982 (1994)). Following homogenization, the homogenates are centrifuged at 4°C for 5 minutes, to separate supernate from debris. For protein quantitation, 3-10 μl of supernate are added to 1.5 ml of bicinchoninic acid reagent (Sigma, St. Louis, MO), and the resulting absorbance at 562 nm is measured.

For SDS-PAGE, samples are adjusted to desired protein concentration with Tricine Buffer (Novex, San Diego, CA), mixed with an equal volume of 2X Tricine sample buffer (Novex, San Diego,CA), and heated for 5 minutes at 100¡C in a thermal cycler. Samples are then applied to a Novex 10-20% Precast Tricine Gel for electrophoresis. Following electrophoresis, samples are transferred from the gels to nitrocellulose membranes in Novex Tris-Glycine Transfer buffer. Membranes are then probed with specific anti-infective lymphocyte antibodies using the reagents and procedures provided in the Western Lights or Western Lights Plus (Tropix, Bedford, MA) chemiluminesence detection kits. Chemiluminesent bands are visualized by exposing the developed membranes to Hyperfilm ECL (Amersham, Arlington Heights, IL).

Competition experiments are carried out in an analogous manner as above, with the following exception; the primary antibodies (anti-infective lymphocyte polyclonal antisera) are pre-incubated for 30 minutes at room temperature with varying concentrations of non-infective lymphocyte immunogen prior to exposure to the nitrocellulose filter. Development of the Western is performed as above.

After visualization of the bands on film, the bands can also be visualized directly on the membranes by the addition and development of a chromogenic substrate such as 5-bromo-4-chloro-3-indolyl phosphate (BCIP). This chromogenic solution contains 0.016% BCIP in a solution containing 100 mM NaCl, 5 mM $MgCl_2$ and 100 mM Tris-HCl (pH 9.5). The filter is incubated in the solution at room temperature until the bands develop to the desired intensity. Molecular mass determination is made based upon the mobility of pre-stained molecular weight standards (Novex, San Diego, CA) or biotinylated molecular weight standards (Tropix, Bedford, MA).

**Example 7 (comparative)**

EIA Microtiter Plate Assay

The immunoreactivity of antiserum preferably obtained from rabbits or mice as described in Example 4 is determined by means of a microtiter plate EIA, as follows. Protein from infectious or non-infectious lymphocyte preparations as described above (EXAMPLE 2) is prepared by homogenization of lymphocytes in an appropriate buffer for example PBS (7.2) or with a mild detergent such as 0.01% Triton X 100. Next, 100 μl of the above protein solution is placed in each well of an Immulon 2® microtiter plate (Dynex Technologies, Chantilly, VA). The plate is incubated overnight at room temperature and then washed four times with deionized water. The wells are blocked by adding 125 μl of a suitable protein blocking agent, such as Superblock® (Pierce Chemical Company, Rockford, IL), in phosphate buffered saline (PBS, pH 7.4) to each well and then immediately discarding the solution. This blocking procedure is performed three times. Antiserum obtained from immunized rabbits or mice prepared as previously described is diluted in a protein blocking agent (e.g., a 3% Superblock® solution) in PBS containing 0.05% Tween-20® (monolaurate polyoxyethylene ether) (Sigma Chemical Company, St. Louis, MO) and 0.05% sodium azide at dilutions of 1:500, 1:2500, 1:12,500, 1:62,500 and 1:312, 500 and placed in each well of the coated microtiter plate. The wells then are incubated for three hours at room temperature. Each well is washed four times with deionized water. One hundred μl of alkaline phosphatase-conjugated goat anti-rabbit IgG or goat anti-mouse IgG antiserum (Southern Biotech, Birmingham, AB), diluted 1:2000 in 3% Superblock® solution in phosphate buffered saline containing 0.05% Tween 20® and 0.05% sodium azide, is added to each well . The wells are incubated for two hours at room temperature. Next, each well is washed four times with deionized water. One hundred microliters (100 μl) of paranitrophenyl phosphate substrate (Kirkegaard and Perry Laboratories, Gaithersburg, MD) then is added to each well. The wells are incubated for thirty minutes at room temperature. The absorbance at 405 nm is read of each well. Positive reactions are identified by an increase in absorbance at 405 nm in the test well above that absorbance given by a non-immune serum (negative control). A positive reaction is indicative of the presence of detectable anti-infective lymphocyte antibodies.

In addition to titers, apparent affinities [$K_d$(app)] may also be determined for some of the antisera. EIA microtiter plate assay results can be used to derive the apparent dissociation constants ($K_d$) based on an analog of the Michaelis-Menten equation (V. Van Heyningen, Methods in Enzymology, Vol. 121, p. 472 (1986) and further described in X. Qiu, et al, Journal of Immunology, Vol. 156, p. 3350 (1996)):

$$[Ag\text{-}Ab] = [Ag\text{-}Ab]_{max} \quad X \quad \frac{[Ab]}{[Ab] = K_d}$$

Where [Ag-Ab] is the antigen-antibody complex concentration, $[Ag\text{-}Ab]_{max}$ is the maximum complex concentration, [Ab] is the antibody concentration, and $K_d$ is the dissociation constant. During the curve fitting, the [Ag-Ab] is replaced with the background subtracted value of the $OD_{405nm}$ at the given concentration of Ab. Both $K_d$ and $[OD_{405nm}]_{max}$, which corresponds to the, $[Ag\text{-}Ab]_{max,}$ are treated as fitted parameters. The software program Origin can be used for the curve fitting.

**Example 8 (comparative)**

Coating of Solid Phase Particles

A. Coating of Microparticles with Antibodies Which Specifically Bind to Infective Lymphocytes. Affinity purified antibodies which specifically bind to infective lymphocytes (see Example 5) are coated onto microparticles of polystyrene, carboxylated polystyrene, polymethylacrylate or similar particles having a radius in the range of about 0.1 to 20 μm. Microparticles may be either passively or actively coated. One coating method comprises coating EDAC (1-(3-dimethylaminopropyl)-3-ethylcarbodiimide hydrochloride (Aldrich Chemical Co., Milwaukee, WI) activated carboxylated latex microparticles with antibodies which specifically bind to infective lymphocytes , as follows. Briefly, a final 0.375% solid suspension of resin washed carboxylated latex microparticles (available from Bangs) Laboratories, Carmel, IN or Serodyn, Indianapolis, IN) are mixed in a solution containing 50 mM MES buffer, pH 4.0 and 150 mg/l of affinity purified anti-infective lymphocyte antibody (see Example 4) for 15 min in an appropriate container. EDAC coupling agent is added to a final concentration of 5.5 μg/ml to the mixture and mixed for 2.5 h at room temperature.

The microparticles then are washed with 8 volumes of a Tween 20®/sodium phosphate wash buffer (pH 7.2) by tangential flow filtration using a 0.2 μm Microgon Filtration module. Washed microparticles are stored in an appropriate buffer which usually contains a dilute surfactant and irrelevant protein as a blocking agent, until needed.

B. Coating of 1/4 Inch Beads. Antibodies which specifically bind to infective lymphocyte antigen also may be coated on the surface of 1/4 inch polystyrene beads by routine methods known in the art (Snitman et al, US Patent 5,273,882, incorporated herein by reference) and used in competitive binding or EIA sandwich assays.

Polystyrene beads first are cleaned by ultrasonicating them for about 15 seconds in 10 mM $NaHCO_3$ buffer at pH 8.0. The beads then are washed in deionized water until all fines are removed. Beads then are immersed in an antibody solution in 10 mM carbonate buffer, pH 8 to 9.5. The antibody solution can be as dilute as 1 μg/ml in the case of high affinity monoclonal antibodies or as concentrated as about 500 μg/ml for polyclonal antibodies which have not been affinity purified. Beads are coated for at least 12 hours at room temperature, and then they are washed with deionized water. Beads may be air dried or stored wet (in PBS, pH 7.4). They also may be overcoated with protein stabilizers (such as sucrose) or protein blocking agents used as non-specific binding blockers (such as irrelevant proteins, Carnation skim milk, Superblock®, or the like).

**Example 9 (comparative)**

Microparticle Enzyme Immunoassay (MEIA)

Infective lymphocyte antigens are detected in patient test samples by performing a standard antigen competition EIA or antibody sandwich EIA and utilizing a solid phase such as microparticles (MEIA). The assay can be performed on an automated analyzer such as the IMx® Analyzer (Abbott Laboratories, Abbott Park, IL).

A. Antibody Sandwich EIA. Briefly, samples suspected of containing infective lymphocyte antigen are incubated in the presence of anti-infective lymphocyte antibody-coated microparticles (prepared as described in Example 8) in order to form antigen/antibody complexes. The microparticles then are washed and an indicator reagent comprising an antibody conjugated to a signal generating compound (i.e., enzymes such as alkaline phosphatase or horseradish peroxidase) is added to the antigen/antibody complexes or the microparticles and incubated. The microparticles are washed and the bound antibody/antigen/antibody complexes are detected by adding a substrate (e.g., 4-methyl umbelliferyl phosphate (MUP), or OPD/peroxide, respectively), that reacts with the signal generating compound to generate a measurable signal. An elevated signal in the test sample, compared to the signal generated by a negative control, detects the presence of infective lymphocyte antigen. The presence of infective lymphocyte antigen in the test sample is indicative of a diagnosis of transmissible spongiform encephalopathy (TSE).

B. Competitive Binding Assay. The competitive binding assay uses a protein or proteins from an infective lymphocyte preparation that generates a measurable signal when the labeled protein is contacted with an anti-infective lymphocyte antibody coated microparticle. This assay can be performed on the IMx® Analyzer (available from Abbott Laboratories, Abbott Park, IL). The labeled proteins from an infective lymphocyte preparation are added to the infective lymphocyte antibody-coated microparticles (prepared as described in Example 17) in the presence of a test sample suspected of containing infective lymphocyte antigen, and incubated for a time and under conditions sufficient to form labeled infective lymphocyte protein / bound antibody complexes and/or patient infective lymphocyte antigen / bound antibody complexes. The infective lymphocyte antigen in the test sample competes with the labeled infective lymphocyte proteins for binding sites on the microparticle. Infective lymphocyte antigen in the test sample results in a lowered binding of labeled infective lymphocyte protein and antibody coated microparticles in the assay since antigen in the test sample and the infective lymphocyte protein compete for antibody binding sites. A lowered signal (compared to a control) indicates the presence of infective lymphocyte antigen in the test sample. The presence of infective lymphocyte antigen suggests the diagnosis of TSE.

The infective lymphocyte proteins discussed hereinabove are useful as markers of TSE. Tests based upon the appearance of this marker or markers in a test sample such as blood, serum, plasma, cerebral spinal fluid, and tissues can provide low cost, non-invasive, diagnostic information to aid the physician to make a diagnosis of TSE, to help select a therapy protocol, or to monitor the success of a chosen therapy. This marker or markers may appear in readily accessible body fluids such as blood, urine, CSF, or stool as antigens derived from the diseased tissue which are detectable by immunological methods. This marker may be elevated in a disease state, altered in a disease state, or be a normal protein which appears in an inappropriate body compartment, in an altered state or form indicative of disease.

References cited in the present Application

1. Hill, A. F. et al. The same prion strain causes vCJD and BSE. Nature 389, 448-450 (1997).
2. Bruce, M. E. et al. Transmissions to mice indicate that 'new variant' CID is caused by the BSE agent. Nature 389, 498-501 (1997).
3. Kitamoto, T., Muramoto, T., Mohri, S., Dohura, K. & Tateishi, J. Abnormal isoform of prion protein accumulates in follicular dendritic dells in mice with Creutzfeldt-Jakob disease. J. Virol. 65, 6292-6295 (1991).
4. Lasmezas, C. I. et al. Immune system-dependent and-independent replicaiton of the scrapie agent: J. Virol. 70, 1292-1295 (1996).
5. Shinkai, Y. et al. RAG-2-deficient mice lack mature lymphocytes owing to inability to initiate V(D)J rearrangement. Cell 68, 855-867 (1992).
6. Mombaerts, P. et al. RAG-1-deficient mice have no mature B and T lymphocytes. Cell 68, 869-877 (1992).
7. Huang, S. et al. Immune response in mice that lack the interferon-y receptor. Science 259, 1742-1745 (1993).
8. Müller, U. et al. Functional role of type I and type II interferons in antiviral defense. Science 264, 1918-1921 (1994).
9. Rahemtulla, A. et al. Normal development and function of CD8+ cells but markedly decreased helpercell activity in mice lacking CD4. Nature 353, 180-184 (1991).
10. Fung Leung, W. P. et al. CD8 is needed for development of cytotoxic T cells but not helper T cells. Cell 65, 143-449 (1991).
11. Ziilstra, M. et al. β 2-Microgiobuin-deficient mice lack CD4+8+ cytolytic T cells. Nature 344, 742-746 (1990).
12. Kägi, D. et al. Cytotoxicity mediated by T cells and natural killer cells is greatly impaired in perforindeficient mice, Nature 369, 31-37 (1994).
13. Kitamura, D., Roes, J., Kuhn, R. & Rajewsky, K. A B-cell-deficient mouse by targeted disruption of the membrane exon of the immunoglobulin Mu-chain gene. Nature 350, 423-426 (1991).
14. Fischer, M. et al. Prion protein (PrP) with amino-proximal deletions rstoring susceptibility of PrP-knockout mice to scrapie. EMBO J. 15, 1255-1264 (1996).
15. Büeler, H. R. et al. Normal development and behaviour of mice lacking the neuronal cell-surface PrP protein. Nature 356, 577-582 (1992).
16. Büeler, H. R. et al. Mice devoid of PrP are resistant to scrapie. Cell 73, 1339-1347 (1993).
17. Fraser, H. et al. Replication of scrapie in spleens of scid mice follows reconstitution with wild-type mouse bone marrow. J. Gen. Virol. 77, 1935-1940 (1996).
18. Nonoyama, S., Smith, F. O., Bernstein, I. D. & Ochs, H. D. Strain-dependent leakiness of mice with severe combined immune deficiency. J. Immunol. 150, 3817-3824 (1993).
19. Bosma, M. J. & Carroll, A. M. The SCID mouse mutant: definition, characterization, and potential uses. Annu. Rev. Immunol. 9, 323-350 (1991).
20. Eigen, M. Prionics or the kinetic basis of prion diseases. Biophys. Chem. 63, A1-18 (1996).
21. Hill, A. F., Zeidler, M., Ironside, J. & Collinge, J. Diagnosis of new variant Creutzfeldt-Jakob disease by tonsil biopsy. Lancet 349, 99 (1997).
22. Rothe, J. et al. Mice lacking the tumour-necrosis factor receptor 1 are resistant to TNF-mediated toxicity but highly susceptible to infection by Listeria monocytogenes. Nature 364, 798-802 (1993).
23. Le Hir, M. et al. Differentiation of follicular dendritic cells and full antibody responses require tumor necrosis factor receptor-1 signaling. J. Exp. Med. 183, 2367-2372 (1996).
24. Humphrey, J. H., Grennan, D. & Sundaram; V. The origin of follicular dendritic cells in the mouse and the mechanism of trapping of immune complexes on them. Eur. J. Immunol. 14, 859-864 (1984).
25. Blättler, T. et al. Transfer of scrapie infectivity from spleen to brain depends on interposed PrP-expressing tissue. Nature 389, 69-73 (1997).
26. Farquhar, C.F., Somerville, R. a. & Ritchie, L. A. Postmortem immunodiagnesis of scrapie and bovine spongiform encephalopathy. J. Virol. Meth. 24, 215-221 (1989).
27. Kalinke, U. et al. The role of somatic mutation in the generation of the protective humoral immune response against vesicular stomatitis virus. Immunity 5, 639-652 (1996).
28. Prusiner, S. B. et al. Measurement of the scrapie agent using an incubation time interval assay. Neurol. 11, 353-358 (1982).
29. Brandner, S. et al. Normal host prion protein (PrPc) required for scrapie spread within the central nervous system. Proc. Natl Acad. Sci. USA 93, 13148-13151 (1996).


Above listed references as well as further references (articles or Patents) cited in the specification as above are hereby included by reference to the disclosure of the present application.

## Claims

1. **1.** Assay method for the determination of the presence of transmissible spongiform encephalopathy in body fluid or tissue derived products isolated from humans or animals, **characterized in that** said method comprises the steps of

   a) collecting the B cells and/or the T cells from a test sample
   b) testing one or both cell types for the presence of transmissible spongiform encephalopathy by inoculating said cells into the cerebrum of a non-human test animal, wherein the development of transmissible spongiform encephalopathy in said test animal indicates the presence of said pathology in the body fluids or tissue derived products from which the tested cells were extracted.

**2.** Assay method for the determination of the presence of transmissible spongiform encephalopathy according to claim 1, wherein the cells collected and tested are B-cells.

**3.** Assay method for the determination of the presence of transmissible spongiform encephalopathy according to claim 1, wherein the cells collected and tested are T-cells.

**4.** Assay method for monitoring of the progress of transmissible spongiform encephalopathy in humans or animals, **characterized in that** the assay method of claim 1 is carried out by collecting and testing both B-cells and T-cells.

**5.** Assay method according to claim 4, **characterized in that** the B-cells are tested first for infection and, if they are found to be infected, then the T-cells are tested for infection.

## Patentansprüche

**1.** Assayverfahren für die Bestimmung der Anwesenheit von übertragbarer spongiformer Encephalopathie in von Körperflüssigkeiten oder -gewebe abgeleiteten Produkten, die aus Menschen oder Tieren isoliert werden, **dadurch gekennzeichnet, dass** das Verfahren die folgenden Schritte umfasst:

a) Sammeln der B-Zellen und/oder der T-Zellen von einer Testprobe
b) Testen von einem oder beiden Zelltypen auf die Anwesenheit von übertragbarer spongiformer Encephalopathie durch Inokuliegen dieser Zellen in das Großhirn eines nicht menschlichen Testtieres, worin die Entwicklung von übertragbarer spongiformer Encephalopathie in dem Testtier das Vorhandensein der Pathologie in den von Körperflüssigkeiten oder -gewebe abgeleiteten Produkten anzeigt, aus welchen die getesteten Zellen extrahiert wurden.

**2.** Assayverfahren für die Bestimmung der Anwesenheit von übertragbarer spongiformer Encephalopathie gemäß Anspruch 1, worin die gesammelten und getesteten Zellen B-Zellen sind.

**3.** Assayverfahren für die Bestimmung der Anwesenheit von übertragbarer spongiformer Encephalopathie gemäß Anspruch 1, worin die gesammelten und getesteten Zellen T-Zellen sind.

**4.** Assayverfahren zur Überwachung des Fortschreitens von übertragbarer spongiformer Encephalopathie in Menschen oder in Tieren, **dadurch gekennzeichnet, dass** das Assayverfahren von Anspruch 1 durch Sammeln und Testen von sowohl B-Zellen als auch T-Zellen ausgeführt wird.

**5.** Assayverfahren gemäß Anspruch 4, **dadurch gekennzeichnet, dass** die B-Zellen zuerst auf Infektion hin getestet werden, und wenn sie als infiziert gefunden werden, dann die T-Zellen auf Infektion hin getestet werden.

## Revendications

**1.** Procédé de dosage destiné à déterminer la présence d'une encéphalopathie spongiforme transmissible dans des produits dérivés de liquides ou de tissus corporels isolés d'humains ou d'animaux, **caractérisé en ce que** ledit procédé comprend les étapes de :

a) recueil des lymphocytes B et/ou des lymphocytes T à partir d'un échantillon à tester

b) détermination dans l'un des types de lymphocytes ou les deux de la présence d'une encéphalopathie spongiforme transmissible par inoculation desdits lymphocytes dans le cerveau d'un animal à tester non humain, où le développement d'une encéphalopathie spongiforme transmissible chez ledit animal à tester indique la présence de ladite pathologie dans les produits dérivés des liquides ou des tissus corporels d'où les lymphocytes testés ont été extraits.

**2.** Procédé de dosage destiné à déterminer la présence d'une encéphalopathie spongiform transmissible selon la revendication 1, où les lymphocytes recueillis et testés sont des lymphocytes B.

**3.** Procédé de dosage destiné à déterminer la présence d'une encéphalopathie spongiforme transmissible selon la revendication 1, où les lymphocytes recueillis et testés sont des lymphocytes T.

**4.** Procédé de dosage destiné à surveiller la progression d'une encéphalopathie spongiforme transmissible chez les humaines ou les animaux, **caractérisé en ce que** le procédé de dosage selon la revendication 1 est mis en oeuvre par recueil et test des lymphocytes B et des lymphocytes T.

**5.** Procédé de dosage selon la revendication 4, **caractérisé en ce que** l'on recherche d'abord l'infection chez les lymphocytes B et, si on constate qu'ils sont infectés, on recherche ensuite l'infection chez les lymphocytes T.

FIG. 1

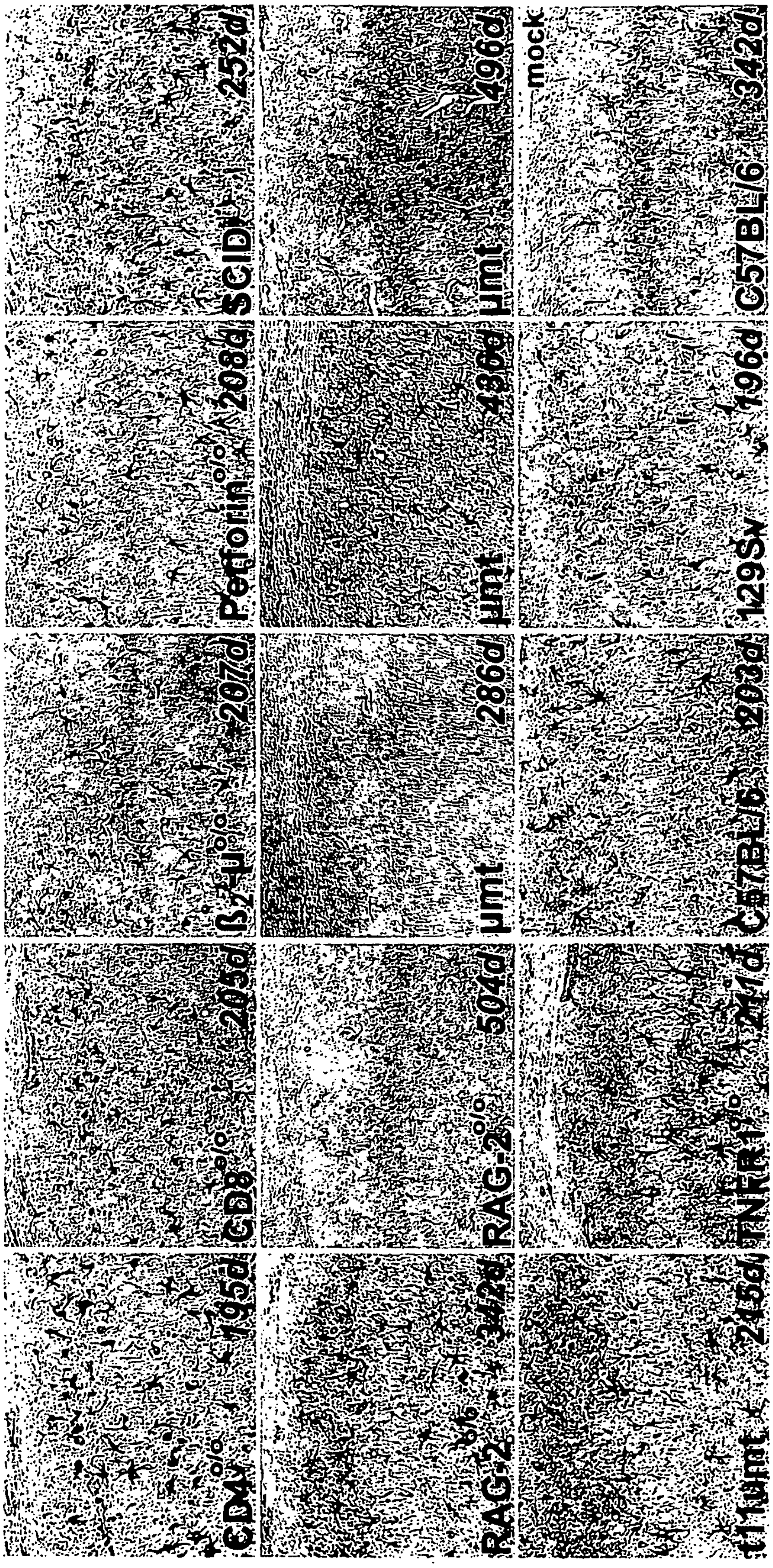
CD4°/° 195d
CD8°/° 205d
207d
Perforin°/° 208d
SCID 252d
RAG-2°/° 342d
RAG-2°/° 504d
μmt 286d
μmt 490d
μmt 496d
TNFR1°/° 211d
C57BL/6 203d
129Sv 196d
C57BL/6 342d
mock

FIG. 2a

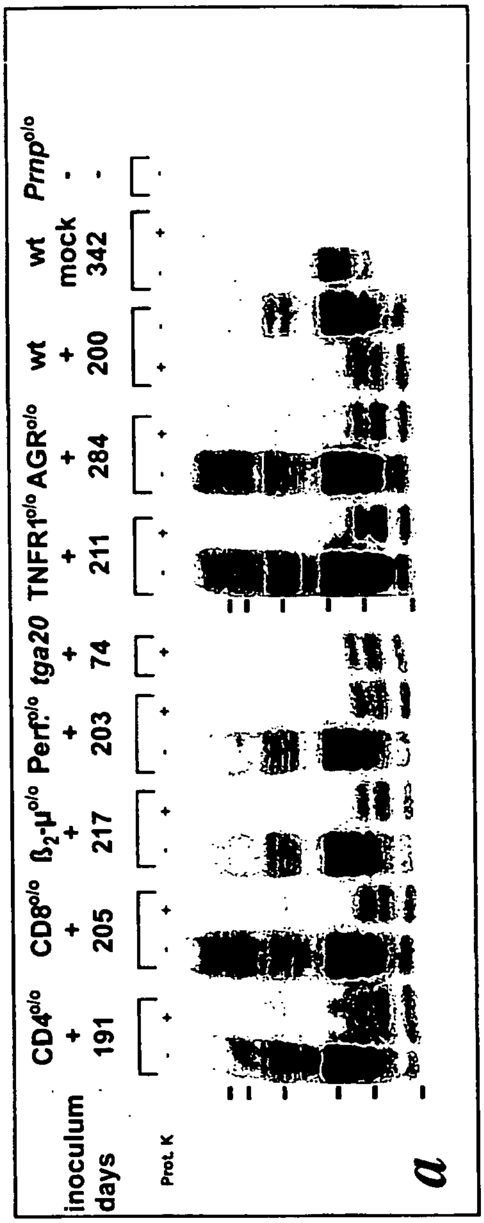
inoculum
days
Prot. K
CD4o/o + 191
CD8o/o + 205
ß2-μo/o + 217
Perf.o/o + 203
tga20 + 74
TNFR1o/o + 211
AGRo/o + 284
wt + 200
wt mock 342
Prnpo/o - -
a

FIG. 2b

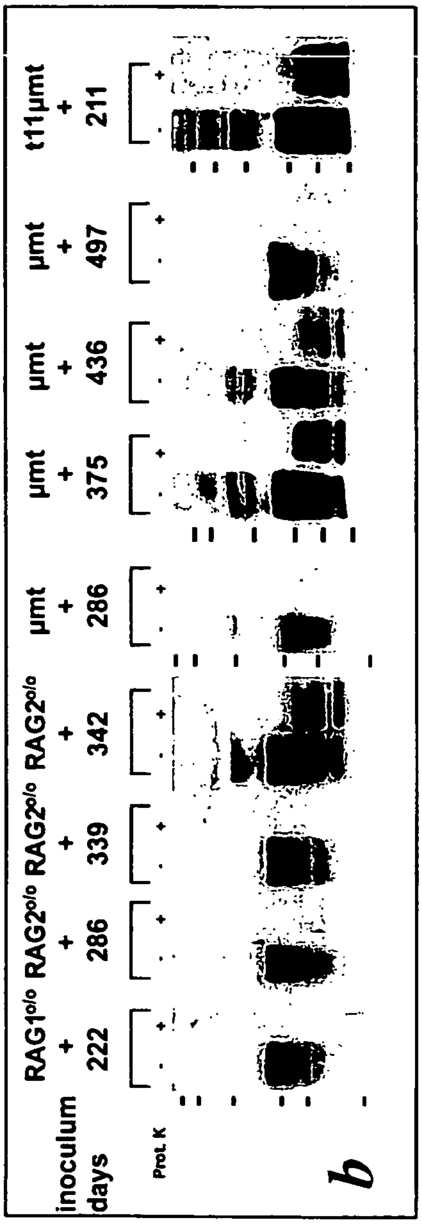
inoculum
days
Prot. K
RAG1o/o
+
222
RAG2o/o
+
286
RAG2o/o
+
339
RAG2o/o
+
342
µmt
+
286
µmt
+
375
µmt
+
436
µmt
+
497
t11µmt
+
211
b

FIG. 2c

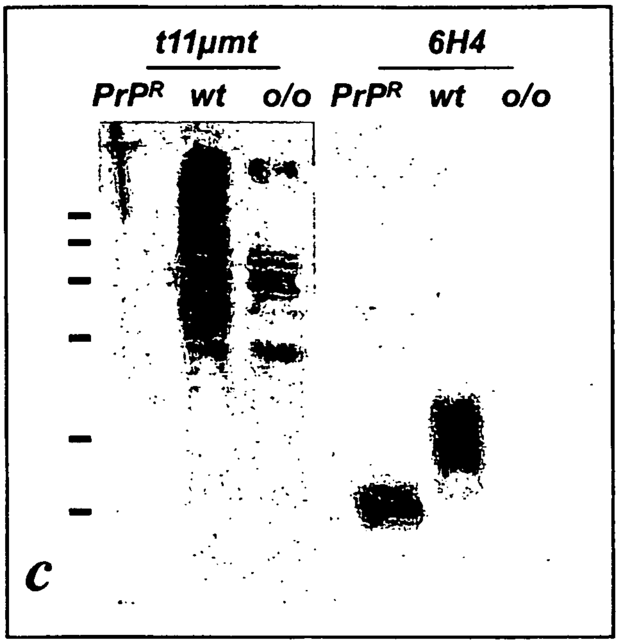

FIG. 2d

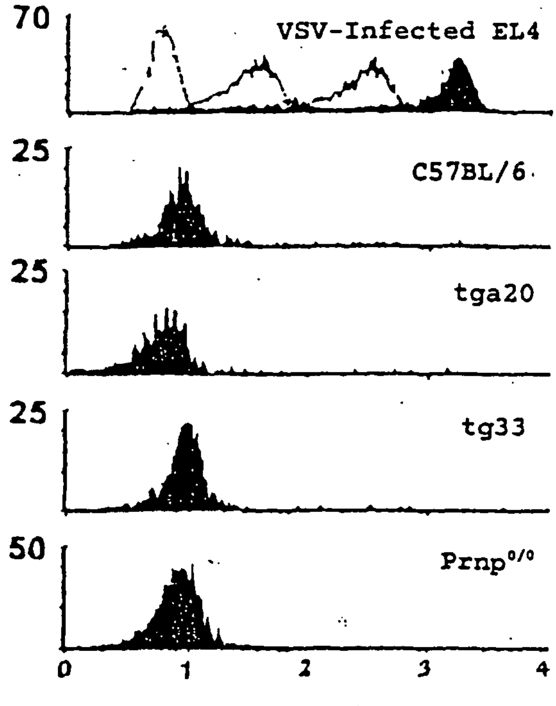
70
VSV-Infected EL4
25
C57BL/6
25
tga20
25
tg33
50
Prnp0/0
0
1
2
3
4

FIG. 3a

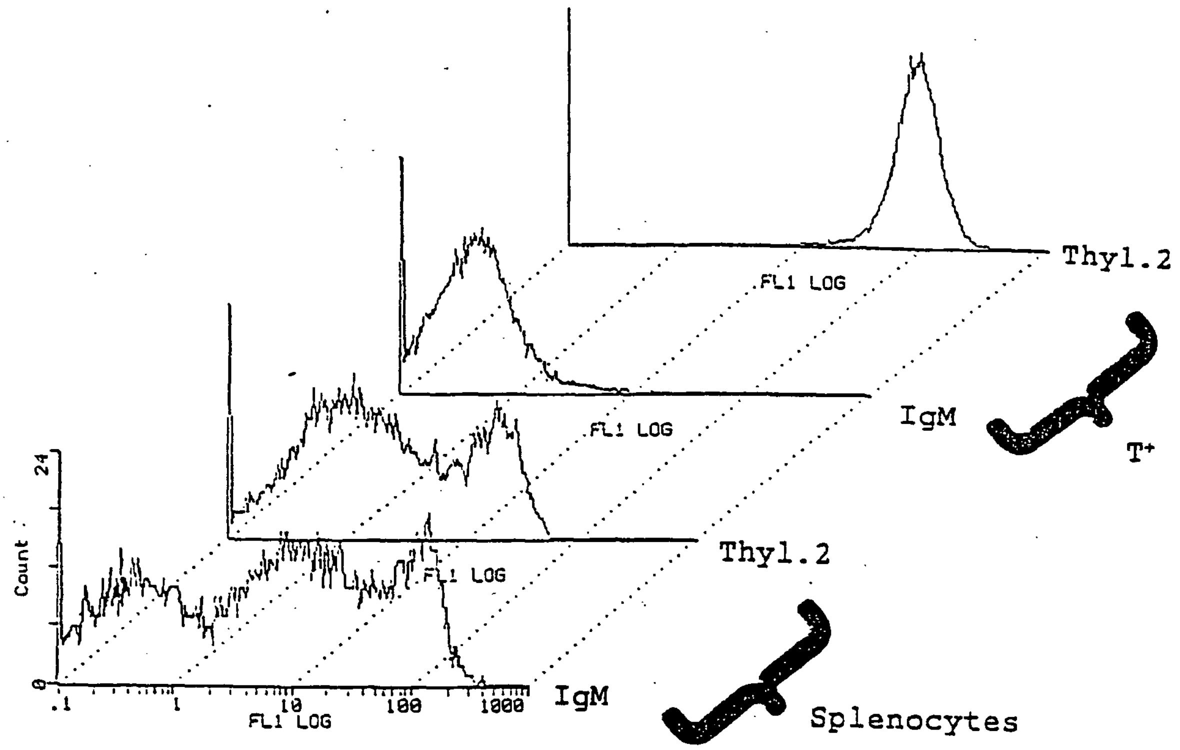
Thyl.2
FL1 LOG
IgM
T+
FL1 LOG
Thyl.2
24
Count
0
FL1 LOG
.1
1
10
100
1000
FL1 LOG
IgM
Splenocytes

FIG. 3b

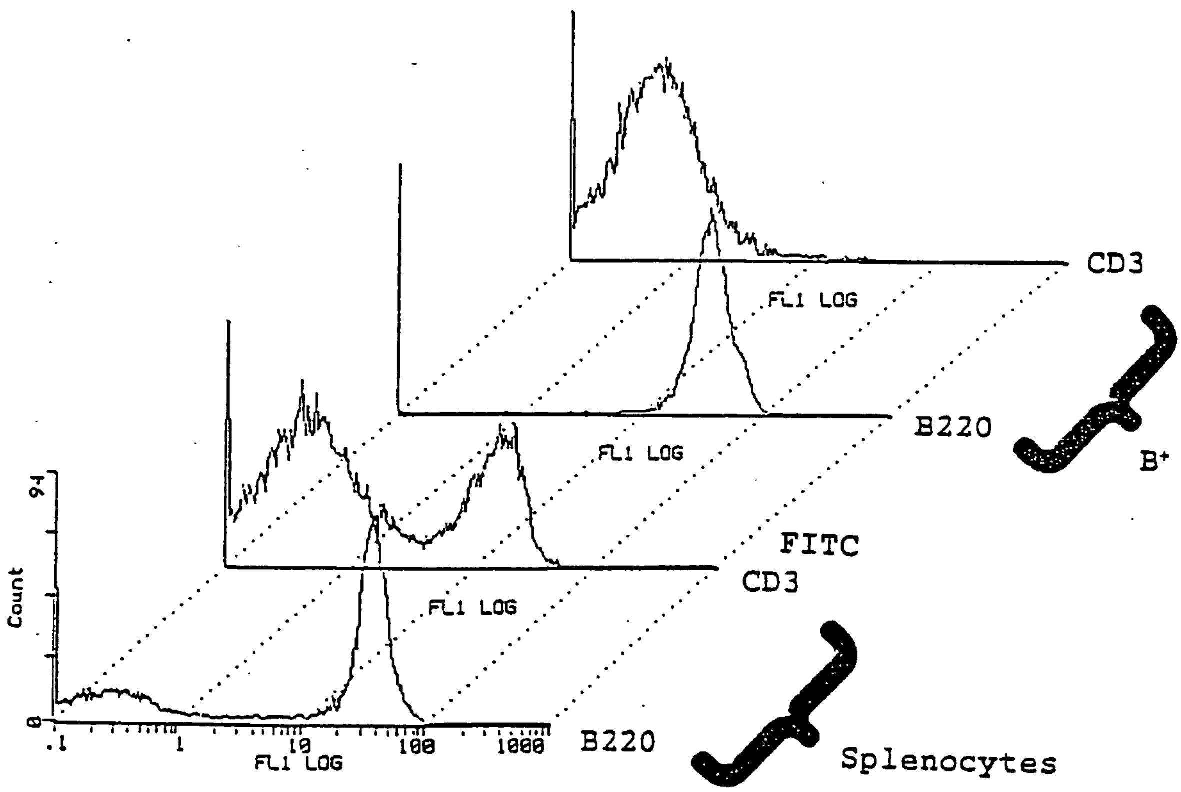
CD3
FL1 LOG
B220
B+
FITC
CD3
FL1 LOG
94
Count
0
.1
1
10
100
1000
FL1 LOG
B220
Splenocytes

Fig. 4

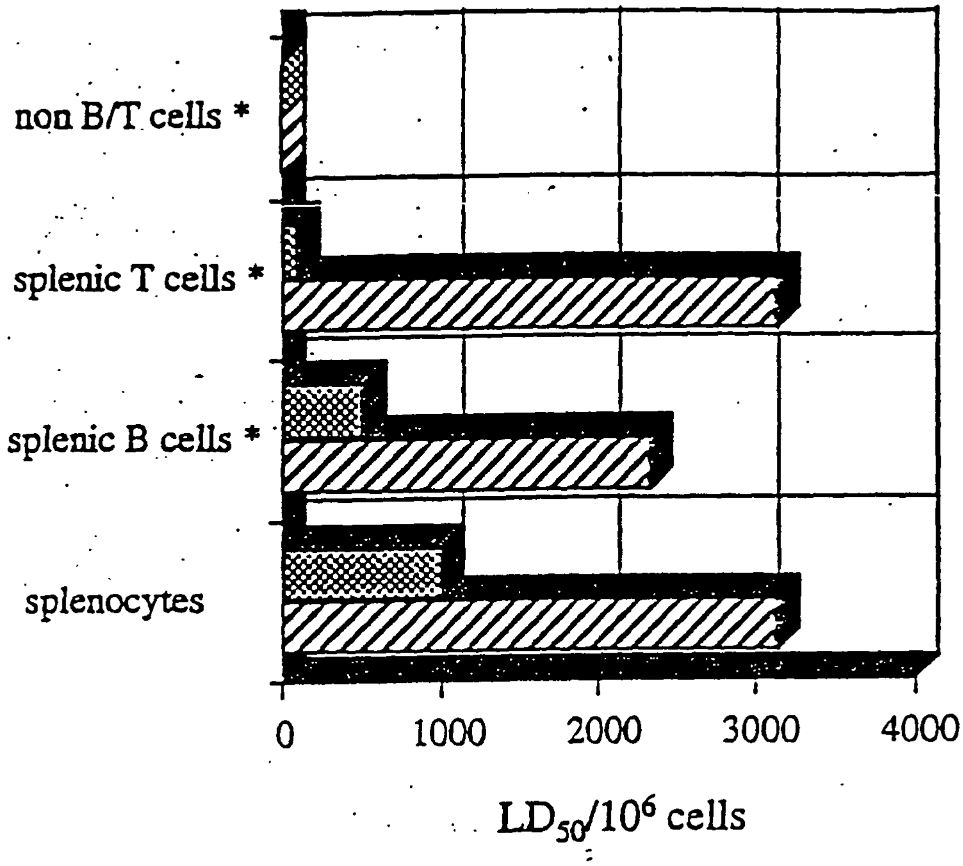


* B and T cells enriched or depleted by magnetic activated cell sorting (MACS) and complement lysis.

Wild type mice

"Spleen mice"

FIG. 5a

Wild-type mice

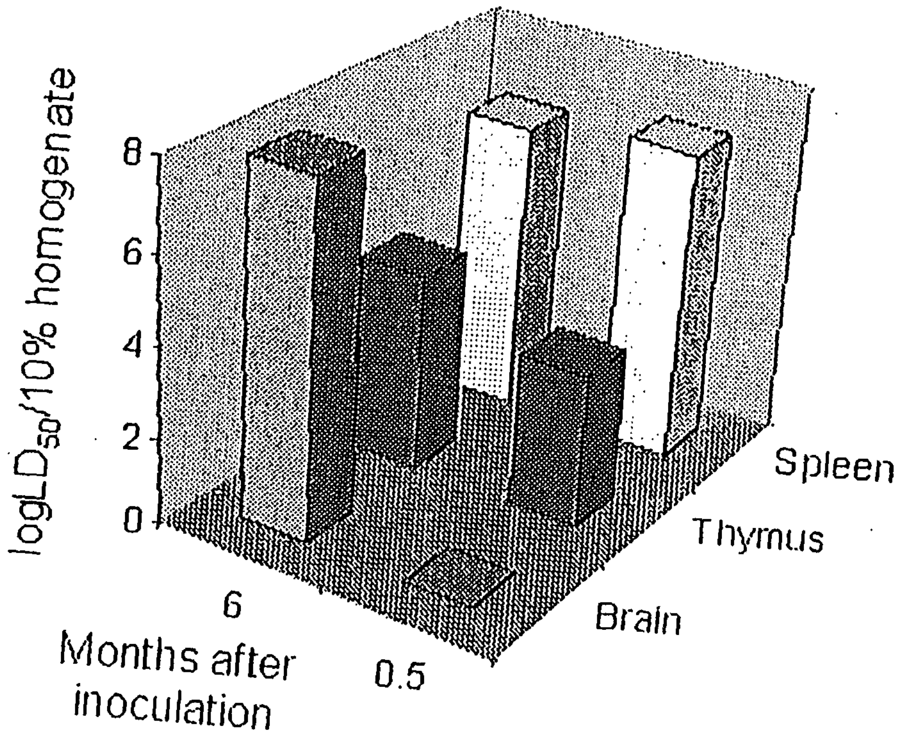

FIG. 5b

"T cell mice"

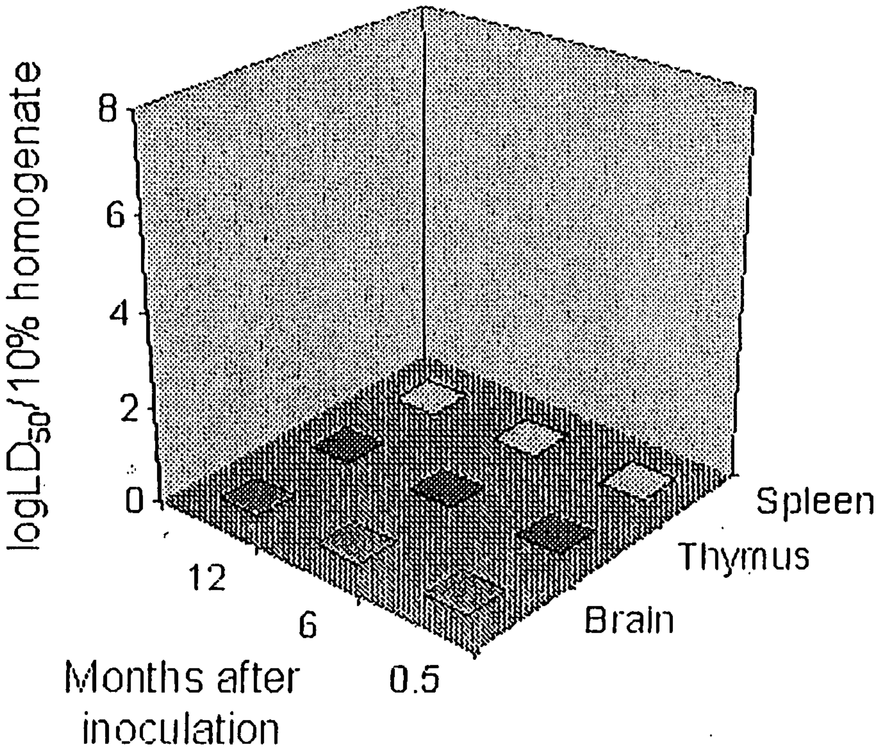

"Spleen mice"

FIG. 5c

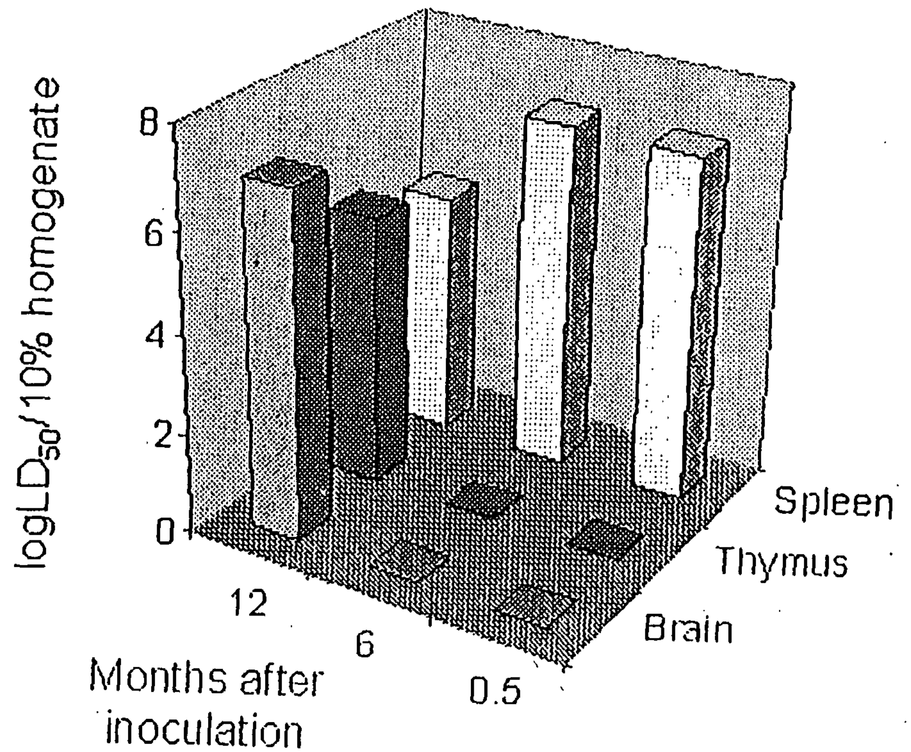

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5273882 A, Snitman


### Non-patent literature cited in the description


- **PRUSINER, S.B.** Novel proteinaceous infectious particles cause scrapie. *Science,* 1982, vol. 216, 136-144
- **GORDON, W.S.** *Vet Rec,* 1946, vol. 58, 516
- **PATTISON, I.H.** Resistance of the scrapie agent to formalin. *J comp Pathol,* 1965, vol. 75, 159-164
- **ALPER et al.** The exceptionally small size of the scrapie agent. *Biochem. Biophys. Res. Commun.,* 1966, vol. 22, 278-284
- **LATARJET et al.** Inactivation of the scrapie agent by near monochromatic ultraviolet light. *Nature,* 1970, vol. 227, 1341-1343
- **ALPER et al.** Does the agent of scrapie replicate without nucleic acid?. *Nature,* 1967, vol. 214, 764-766
- **GIBBON, R.A. ; HUNTER, G.D.** Nature of the scrapie agent. *Nature,* 1967, vol. 215, 1041-1043
- **PATTISON, I.H. ; JONES, K.M.** The possible nature of the transmissible agent of scrapie. *Vet. Rec.,* 1967, vol. 80, 2-9
- **PRUSINER, S.B. ; HSIAO, K.K.** Human prion diseases. *Ann. Neurol.,* 1994, vol. 35, 385-395
- **WEISSMANN, C.** Molecular biology of prion diseases. *Trends Cell Biol.,* 1994, vol. 4, 10-14
- **OESCH et al.** A cellular gene encodes scrapie PrP 27-30 Protein. *Cell,* 1985, vol. 40, 735-746
- **CHESEBRO et al.** Identification of scrapie prion protein-specific mRNA in scrapie-infected and uninfected brain. *Nature,* 1985, vol. 315, 331-333
- **MANSON et al.** The prion protein gene: a role in mouse embryogenesis?. *Development,* 1992, vol. 115, 117-122
- **BENDHEIM et al.** Nearly ubiquitous tissue distribution of the scrapie agent precursor protein. *Neurology,* 1992, vol. 42, 149-156
- **MC KINLEY et al.** Scrapie prion rod formation in vitro requires both detergent extraction and limited proteolysis. *J. Vitrol.,* 1991, vol. 65, 1340-1351
- **STAHL et al.** Structural studies of the scrapie prion protein using mass spectrometry and amino acid sequencing. *Biochemistry,* 1993, vol. 32, 1991-2002
- **OESCH et al.** Search for a scrapie-specific nucleic acid: a progress report. *Ciba. Found. Symp.,* 1988, vol. 135, 209-223
- **PRUSINER et al.** Transgenetic studies implicate interactions between homologous PrP isoforms in scrapie prion replication. *Cell,* 1990, vol. 63, 673-686
- **BOLTON, D.C. ; BENDHEIM, P.E.** A modified host protein model of scrapie. *Ciba. Found. Symp.,* 1988, vol. 135, 164-181
- **DICKINSON, A.G. ; OUTRAM, G.W.** Genetic aspects of unconventional virus infections: the basis of the virino hypothesis. *Ciba. Found. Symp.,* 1988, vol. 135, 63-83
- **HOPE, J.** The nature of the scrapie agent: the evolution of the virino. *Ann. N. Y. Acad. Sci.,* 1994, vol. 724, 282-289
- **DIRINGER et al.** The nature of the scrapie agent: the virus theory. *Ann. N. Y. Acad. Sci.,* 1994, vol. 724, 246-258
- **POCCHIARI, M.** Prions and related neurological diseases. *Molec. Aspects. Med.,* 1994, vol. 15, 195-291
- **ROHWER, R.G.** The scrapie agent: ''a virus by any other name. *Curr. Top. Microbiol. Immunol.,* 1991, vol. 172, 195-232
- **KELLINGS et al.** Futher analysis of nucleic acids in purified scrapie prion preparations by improved return refocusing gel electrophoresis. *J. Gen. Virol.,* 1992, vol. 73, 1025-1029
- **BOLTON et al.** Identification of a protein thath purifies with the scrapie prion. *Science,* 1982, vol. 218, 1309-1311
- **BASLER et al.** Scrapie and cellular PrP isoforms are encoded by the same chromosomal gene. *Cell,* 1986, vol. 46, 417-428
- **HSIAO et al.** Linkage of a prion protein missense variant to Gerstmann-Straussler syndrome. *Nature,* 1989, vol. 338, 342-345
- **CAUGHEY et al.** Secondary structure analysis of the scrapie-associated protein PrP 27-30 in water by infrared spectroscopy. *Biochemistry,* 1991, vol. 30, 7672-7680

- **COHEN et al.** Structural clues to prion replication. *Science,* 1994, vol. 264, 530-531
- **HUANG et al.** Proposed three-dimensional structure for the cellular prion protein. *Proc. Natl. Acad. Sci. U.S.A.,* 1994, vol. 91, 7139-7143
- **PAN et al.** Conversion of alpha-helices into beta-sheets features in the formation of the scrapie prion proteins. *Proc. Natl. Acad. Sci. U.S.A.,* 1993
- **SAFAR et al.** Conformational transitions, dissociation, and unfolding of scrapie amyloid (prion) protein. *J. Biol. Chem.,* 1993, vol. 268, 20276-20284
- **EKLUND et al.** Pathogenesis of scrapie virus infection in the mouse. *J. infect. Dis.,* 1967, vol. 117, 15-22
- **CLARKE, M.C. ; KOMBERLIN, R.H.** Pathogenesis of mouse scrapie: distribution of agent in the pulp and stroma of infected spleens. *Vet. Microbiol.,* 1984, vol. 9, 215-225
- **FRASER, H. ; DICKINSON, A.G.** Studies of the lymphoreticular system in the pathogenesis of scrapie: the role of spleen and thymus. *J. Comp. Pathol.,* 1978, vol. 88, 563-573
- **KIMBERLIN, R.H. ; WALKER, C.A.** Pathogenesis of experminetal scrapie. *Ciba. Found. Symp.,* 1988, vol. 135, 37-62
- **RUBENSTEIN et al.** Scrapie-infected spleen: analysis of infectivity, scrapie-associated fibrils, and protease-resistant proteins. *J. infect. Dis.,* 1991, vol. 164, 29-35
- **FRASER et al.** The scrapie disease process is unaffected by ionising radiation. *Prog. Clin. Biol. Res.,* vol. 317, 653-658
- **MURAMOTO et al.** Species barrier prevents an abnormal isoform of prion protein from accumulating in follicular dendritic cells of mice with Creutzfeldt-Jakob disease. *J. Virol.,* 1993, vol. 67, 6808-6810
- **BÜELER et al.** *Cell,* 1993, vol. 73, 1339-1347
- **CHAFFIN et al.** *EMBO J.,* vol. 9, 3821-3829
- **YAMADA et al.** *Proc.Natl.Acad.Sci.USA.,* 1991, vol. 88, 532-536
- **BRANDNER et al.** Normal host prion protein necessary for scrapie-induced neurotoxicity. *Nature,* 1996, vol. 379
- **KOHLER ; MILSTEIN.** *Nature,* 1975, vol. 256, 494
- Monoclonal Hybridoma Antibodies: Techniques and Applications. CRC Press, Inc, 1982
- **L.T. MIMMS et al.** *Virology,* 1990, vol. 176, 604-619
- **KAIN et al.** *Biotechniques,* 1994, vol. 17, 982
- **V. VAN HEYNINGEN.** *Methods in Enzymology,* 1986, vol. 121, 472
- **X. QIU et al.** *Journal of Immunology,* 1996, vol. 156, 3350
- **HILL, A. F. et al.** The same prion strain causes vCJD and BSE. *Nature,* 1997, vol. 389, 448-450
- **BRUCE, M. E. et al.** Transmissions to mice indicate that 'new variant' CID is caused by the BSE agent. *Nature,* 1997, vol. 389, 498-501
- **KITAMOTO, T. ; MURAMOTO, T. ; MOHRI, S. ; DOHURA, K. ; TATEISHI, J.** Abnormal isoform of prion protein accumulates in follicular dendritic dells in mice with Creutzfeldt-Jakob disease. *J. Virol.,* 1991, vol. 65, 6292-6295
- **LASMEZAS, C. I. et al.** Immune system-dependent and-independent replicaiton of the scrapie agent. *J. Virol.,* 1996, vol. 70, 1292-1295
- **SHINKAI, Y. et al.** RAG-2-deficient mice lack mature lymphocytes owing to inability to initiate V(D)J rearrangement. *Cell,* 1992, vol. 68, 855-867
- **MOMBAERTS, P. et al.** RAG-1-deficient mice have no mature B and T lymphocytes. *Cell,* 1992, vol. 68, 869-877
- **HUANG, S. et al.** Immune response in mice that lack the interferon-y receptor. *Science,* 1993, vol. 259, 1742-1745
- **MÜLLER, U. et al.** Functional role of type I and type II interferons in antiviral defense. *Science,* 1994, vol. 264, 1918-1921
- **RAHEMTULLA, A. et al.** Normal development and function of CD8+ cells but markedly decreased helpercell activity in mice lacking CD4. *Nature,* 1991, vol. 353, 180-184
- **FUNG LEUNG, W. P. et al.** CD8 is needed for development of cytotoxic T cells but not helper T cells. *Cell,* 1991, vol. 65, 143-449
- **ZIILSTRA, M. et al.** β 2-Microgiobuin-deficient mice lack CD4+8+ cytolytic T cells. *Nature,* 1990, vol. 344, 742-746
- **KÄGI, D. et al.** Cytotoxicity mediated by T cells and natural killer cells is greatly impaired in perforindeficient mice. *Nature,* 1994, vol. 369, 31-37
- **KITAMURA, D. ; ROES, J. ; KUHN, R. ; RAJEWSKY, K.** A B-cell-deficient mouse by targeted disruption of the membrane exon of the immunoglobulin Mu-chain gene. *Nature,* 1991, vol. 350, 423-426
- **FISCHER, M. et al.** Prion protein (PrP) with amino-proximal deletions rstoring susceptibility of PrP-knockout mice to scrapie. *EMBO J.,* 1996, vol. 15, 1255-1264
- **BÜELER, H. R. et al.** Normal development and behaviour of mice lacking the neuronal cell-surface PrP protein. *Nature,* 1992, vol. 356, 577-582
- **BÜELER, H. R. et al.** Mice devoid of PrP are resistant to scrapie. *Cell,* 1993, vol. 73, 1339-1347
- **FRASER, H. et al.** Replication of scrapie in spleens of scid mice follows reconstitution with wild-type mouse bone marrow. *J. Gen. Virol.,* 1996, vol. 77, 1935-1940
- **NONOYAMA, S. ; SMITH, F. O. ; BERNSTEIN, I. D. ; OCHS, H. D.** Strain-dependent leakiness of mice with severe combined immune deficiency. *J. Immunol.,* 1993, vol. 150, 3817-3824

- **BOSMA, M. J. ; CARROLL, A. M.** The SCID mouse mutant: definition, characterization, and potential uses. *Annu. Rev. Immunol.,* 1991, vol. 9, 323-350
- **EIGEN, M.** Prionics or the kinetic basis of prion diseases. *Biophys. Chem.,* 1996, vol. 63, A1-18
- **HILL, A. F. ; ZEIDLER, M. ; IRONSIDE, J. ; COLLINGE, J.** Diagnosis of new variant Creutzfeldt-Jakob disease by tonsil biopsy. *Lancet,* 1997, vol. 349, 99
- **ROTHE, J. et al.** Mice lacking the tumour-necrosis factor receptor 1 are resistant to TNF-mediated toxicity but highly susceptible to infection by Listeria monocytogenes. *Nature,* 1993, vol. 364, 798-802
- **LE HIR, M. et al.** Differentiation of follicular dendritic cells and full antibody responses require tumor necrosis factor receptor-1 signaling. *J. Exp. Med.,* 1996, vol. 183, 2367-2372
- **HUMPHREY, J. H. ; GRENNAN, D. ; SUNDARAM; V.** The origin of follicular dendritic cells in the mouse and the mechanism of trapping of immune complexes on them. *Eur. J. Immunol.,* 1984, vol. 14, 859-864
- **BLÄTTLER, T. et al.** Transfer of scrapie infectivity from spleen to brain depends on interposed PrP-expressing tissue. *Nature,* 1997, vol. 389, 69-73
- **FARQUHAR, C.F. ; SOMERVILLE, R. ; RITCHIE, L. A.** Postmortem immunodiagnesis of scrapie and bovine spongiform encephalopathy. *J. Virol. Meth.,* 1989, vol. 24, 215-221
- **KALINKE, U. et al.** The role of somatic mutation in the generation of the protective humoral immune response against vesicular stomatitis virus. *Immunity,* 1996, vol. 5, 639-652
- **PRUSINER, S. B. et al.** Measurement of the scrapie agent using an incubation time interval assay. *Neurol.,* 1982, vol. 11, 353-358
- **BRANDNER, S. et al.** Normal host prion protein (PrPc) required for scrapie spread within the central nervous system. *Proc. Natl Acad. Sci. USA,* 1996, vol. 93, 13148-13151